# EUROPEAN PATENT SPECIFICATION

(11) **EP 3 923 875 B1**
(45) Date of publication and mention of the grant of the patent: **17.04.2024**
(21) Application number: 20703261.6
(22) Date of filing: 11.02.2020
(51) Int. Cl.: A61F 5/01, A61F 5/32

(54) **ACHILLES TENDON ORTHOSIS**
ACHILLESSEHNENORTHESE
ORTHÈSE POUR TENDON D'ACHILLE

(30) Priority: 12.02.2019 EP 19156596
(43) Date of publication of application: 22.12.2021
(73) Proprietor: Malliaropoulos, Nikolaos G., London W13 0DT (GB); Lohrer, Heinz, 65835 Liederbach (DE); Roller, Matthias, 72336 Balingen (DE)
(72) Inventor: Malliaropoulos, Nikolaos G., London W13 0DT (GB); Lohrer, Heinz, 65835 Liederbach (DE); Roller, Matthias, 72336 Balingen (DE)
(74) Representative: REHBERG HÜPPE + PARTNER
(86) International application number: PCT/EP2020/053454
(87) International publication number: WO 2020/165159

(56) References cited:
- DE-U1-202004 013 159
- US-A1- 2002 029 009
- US-A1- 2006 178 606
- US-B1- 9 421 117
- US-B2- 9 357 804

## Description

### TECHNICAL FIELD OF THE INVENTION

The present invention relates to an Achilles tendon orthosis which can be applied to a foot for treating a symptomatic Achilles tendon or Achilles tendinopathy.

Blood flow in symptomatic tendons was primarily studied in the 1990s with Power Doppler ultrasonography (PDU) (cp. [16]). Later, this blood flow was defined as "neovascularization", confirming that the ingrowth of the new blood vessels into tendinopathic areas of the tendon can often be seen on the ultrasonographic images using Power Doppler or Colour Doppler, indicating the severity of Achilles Tendinopathy (cp. [18]). Chronic painful tendon pathology (tendinopathy) has often been referred to as tendinitis; however it is more accurately described as tendinosis, due to absence of inflammatory processes (cp. [10]). The morphological characteristics of Achilles tendinosis have been shown to be an area with high concentration of glucoseaminoglycans (GAGs) and irregular structure and arrangement, but no inflammatory cells (cp. [15]). The exact pathologic mechanism of the chronic tendinopathy is still unknown; however a possible cause could be a dysbalance in loading, while others suggest excessive vessel and nerve ingrowth as the cause of pain. Some other studies suggest that alterations in histology, histochemistry and biochemistry lead to tendon lesions, hence chronic tendinopathy (cp. [21]). Degenerative tendon disorders are developed where the tendon has a poor, or no blood supply (cp. [20]) and such a typical location is the mid-portion Achilles tendon where the blood vessel density is low.

Achilles tendinopathy is a clear clinical diagnosis based on a combination of pain, swelling, and impaired performance (cp. [13]). It was hypothesized that in symptomatic tendons the induced vasoconstriction might lead to increased blood flow (cp. [1]); while in asymptomatic tendons there may be a physiologically slow blood flow. Signs of increased vascularity have previously been shown in biopsies from patients with chronic Achilles tendon pain (cp. [15]). Additionally, the symptomatic Achilles tendons are characterized by neovascularization. The accompanying nerves are addressed to be the main source of pain especially in chronic midportion Achilles tendinopathy (cp. [1]). In contrast to the previous theory, a recent study examined the relationship between neovascularization and clinical severity in Achilles tendinopathy in 556 paired measurements (cp. [4]) using the Victorian Institute of Sports Assessment-Achilles questionnaire to evaluate the severity of Achilles tendinopathy. No relationship between the amount of pain and degree of neovascularization was found. The differences in the results among these studies is still unknown, however it is believed that there is lack of the uniform manner of assessing the degree of neovascularization. The paratenon of the Achilles tendon is a common location of chronic pain, with marked vascularization and degenerative vascular changes, disturbing blood supply, causing ischemic pain especially during exercise (cp. [11]). In chronic tendinopathy, neovascularization of the tissue is a highly active process. The blood vessels were thought to be formed anew and were described as tortuous vessels with a small lumen, located on the anterior aspect of the Achilles tendon, infiltrating it diffusely. However, the presence of blood vessels was not associated with tissue repair (cp. [2]).

Poor vascularity is associated with limited tendon healing capacity (cp. [8]). The potential effect of PRP injections on neovessels has been well studied (cp. [5], [7], [3]). Several growth factors have been implicated as possible mediators of angiogenesis during wound repair including, growth factor beta-1, epidermal growth factor, platelet derived growth factor (PDGF), and most recently, vascular endothelial growth factor (VEGF) (cp. [9]). It was hypothesized that the growth factors (GFs) could accelerate the process of tendon regeneration, highlighting the fact that it is unlikely that a simple GF would give positive results. However, the interaction of several GFs present in the right concentration at the right time would be beneficial (cp. [12]). An initial increase of neovascularization after PRT injection was reported, but did not differ from what occurred after placebo injection of saline, suggesting a needle instead of content effect (cp. [6]).

High-volume imagine-guided injection (HVIGI) have effectively been used in managing mid-portion Achilles Tendinopathy, demonstrating reduced pain and improved short- and long-term functionality (cp. [14]).

Ultrasound guided sclerosis of neovessels for painful mid-portion chronic Achilles tendinosis was effective in a pilot study (cp. [17]). The widely used agent polidocanol was applied since it had few side effects, was registered drug, and had local anaesthetic effect. The results of this study show that sclerosing the vessels in the pathologically changed Achilles tendons significantly decreased pain and allowed the patients to return to pain free tendon loading activities, suggesting that neovessels could be a cause of pain in chronic mid-portion Achilles tendinosis (cp. [17]).

Eccentric exercise for the treatment of Achilles tendinopathy has been promoted since the mid-1980s (cp. [22]), improving calf muscle strength, stiffening and lengthening the myotenondinus unit, decrease neovascularization, and decrease pain (cp. [19]).

### PRIOR ART

[1] Alfredson H, Ohberg L, Forsgren S (2003) In vasculo-neural ingrowth the cause of pain in chronic Achill tendinosis? An investigation using ultrasonography and color Doppler, immunohistochemistry, and diagnostic injections. Knee Surg Sports Traumatol Arthrosc 11:334-338
[2] Åström M, Rausing A (1995) Chronic Achilles tendinopathy. A survey of surgical and histopathologic findings. Clin Orthop 316:151-164
[3] de Jonge S, de Vos RJ, Weir A, van Schie HT, Bierma-Zeinstra SM, Verhaar JA, Weinans H, Tol JL. (2011) One-year follow-up of platelet-rich plasma treatment in chronic Achilles tendinopathy: a double-blind randomized placebo-controlled trial. Am J Sports Med : 39: 1623-1629.
[4] De Jonge S, Warnaars JL, De Vos RJ, et al. (2013) Relationship between neovascularization and clinical severity in Achilles tendinopathy in 556 paired measurements. Scand J Med Sci Sports. ;24:773-778.
[5] De Vos RJ, Weir A, van Schie HTM, Bierma-Zeinstra SMA, Verhaar JAN, Weinans H, Tol J. (2010) Platelet-rich plasma injection for chronic Achilles tendinopathy: a randomized controlled trial. JAMA : 303: 144-149.
[6] De Vos RJ, Weir A, Tol JL, Verhaar JA, Weinans H, van Schie HT. (2011) No effects of PRP on ultrasonographic tendon structure and neovascularisation in chronic midportion Achilles tendinopathy. Br J Sports Med: 45: 387-392.
[7] Del Buono A, Papalia R, Denaro V, Maccauro G, Maffulli N (2011) Platelet rich plasma and tendinopathy: state of the art. Int J Immunopathol Pharmacol 24(1 Suppl 2):79-83
[8] Fenwick SA, Hazleman BL, Riley GP. (2002) The vasculature and its role in the damaged and healing tendon. Arthritis Res; 4:252-60.
[9] Ferrara, N (2004): Vascular endothelial growth factor: Basic science and clinical progress. Endocr Rev. 25:581 - 611
[10] Kannus P, Jozsa L (1991): Histopathological changes preceding spontaneous rupture of a tendon. A controlled study of 891 patients. J Bone Joint Surg A. 73:1507-1525
[11] Kvist M, Jozsa L, Jarvinen MJ, Kvist H (1987) Chronic Achilles paratenonitis in athletes: a histological and histochemical study. Pathology, 19:1-11
[12] Maffulli N, Kader D. (2002) Tendinopathy of tendo Achillis. J Bone Joint Surg Br; 84:1-8.
[13] Maffulli N, Sharma P, Luscombe KL. (2004) Achilles tendinopathy: aetiology and management. J R Soc Med: 97: 472-476.
[14] Maffulli N, Margiotti K, Longo UG, Loppini M, Fazio VM, Denaro V. (2013) The genetics of sports injuries and athletic performance. Muscles Ligaments Tendons J; 3: 173-189.
[15] Movin T, Gad A, Reinholt FP (1997) Tendon pathology in long-standing Achillodynia. Biopsy findings in 40 patients. Acta Orthop Scand 68:170-175
[16] Newman JS, Adler RS, Bude RO, Rubin JM (1994) Detection of soft-tissue hyperemia: value of power Doppler sonography. AJR Am J Roentgenol 163(2):385-389
[17] Ohberg L, Alfredson H. (2002) Ultrasound guided sclerosis of neovessels in painful chronic Achilles tendinosis: pilot study of a new treatment. Br J Sports Med, 36: 173-175; discussion 176-177.
[18] Ohberg L, Lorentzon R, Alfredson H. (2001) Neovascularisation in Achilles tendons with painful tendinosis but not in normal tendons: an ultrasonographic investigation. Knee Surg Sports Traumatol Arthrosc: 9: 233-238.
[19] Ohberg L, Lorentzon R, Alfredson H (2004) Eccentric training in patients with chronic Achilles tendinosis: normalised tendon structure and decreased thickness at follow up. Br J Sports Med 38(1):8-11
[20] Pufe T, Petersen WJ, Mentlein R, Tillmann BN. (2005) The role of vasculature and angiogenesis for the pathogenesis of degenerative tendons disease. Scand J Med Sci Sport; 15(4):211-222
[21] Riley GP, Harrall RL, Constant CR, Chard MD, Cawston TE, Hazleman BL(1994) Tendon degeneration and chronic shoulder pain: changes in the collagen composition of the human rotator cuff tendons in rotator cuff tendinitis. Ann Rheum. 53:359-366.
[22] Stanish WD, Rubinovich RM, Curwin S. (1986) Eccentric exercise in chronic tendinitis. Clin Orthop Relat Res; 65-68.

Under the labels "ACHILLODYN", "ACHILLO-HIT", "Achimed", "select AchilloStabil Plus", "AchilloTrain" (each registered trademarks) sock-like bandages for the Achilles tendon are distributed. Here, the pressure applied by pelottes to the foot results from the tension of the textile sock (cp. www.sporlastic.de, www.medi.de, www.bort.com).

US 2002/0029009 A1 discloses a rigid ankle and foot orthosis for use in athletic and medical applications for individuals who have injured their ankle or have an unstable ankle joint. The ankle and foot orthosis comprises a lower shell with two upright integral shell parts. A first shell part extends up to a location above the ankle joint whereas the other shell part ends at the ankle joint where it forms a fixable joint with an upright additional shell element. The fixable joint is arranged coaxially with the ankle joint of the wearer and is used for adapting the ankle and foot orthosis to the foot of the wearer. The shell parts of the ankle and foot orthosis are fixed at the foot of the wearer by a plurality of straps. Cushioning pads are arranged between the shell parts and the straps on the one hand and the foot of the wearer on the other hand.

US 9,421,117 B1 discloses an ankle brace for treatment of plantar fasciitis/fasciosis and Achilles tendonitis/tendonosis and more specifically a brace that pneumatically massages soft tissue of the foot for relieving pain and inflammation associated with plantar fasciitis/fasciosis and Achilles tendonitis/tendonosis. The ankle brace comprises a foot plate portion and an ankle portion preferably made of polymethacrylate. The foot plate portion contains a spandex sleeve surrounding the mid-foot of the user and securing the foot to the brace. The sleeve has a distal opening through which the forefoot extends and a proximal opening through which the heel extends. Furthermore, the ankle portion includes a rear portion which covers the posterior portion of the ankle and completely covers the ankle and the Achilles tendon. The rear portion has two slits which accommodate a strap which wraps around the posterior surface. The strap can be fastened by a hook-and-loop fastener. The ankle brace includes a pneumatic air bladder system with an U-shaped air cushion which when properly secured to a foot surrounds the plantar fascia and creates a cushion for the plantar fascia origin at the heel bone and lifts the heel upward. Both ends of the U-shaped air cushion are pneumatically coupled to large air chambers via pneumatic communicating channels. The pneumatic air bladder system provides a pulsatile air flow over the plantar fascia insertion and Achilles tendon during the gait cycle which is accomplished via the unique shape of the bladder and its position on the foot and ankle. Upon heel lift, air from the proximal chambers begin to maximally inflate the distal chamber secondary to the reduction in resistance in the distal chamber. As the foot begins to perform a heel strike, the air begins to compress in the distal chamber and flows to areas of less resistance, namely the proximal chambers. This flow of air causes the chambers to expand and contract thus massaging the underlying body part. The proximal air chambers might contain a durable foam type material seated inside the bladder. The foam material provides memory to the air chambers to add an inflation and lifting of the plantar fascia.

Also US 2006/0178606 A1 discloses the use of inflatable cells for a brace, here for treating posterior tibial tendon dysfunction (PTTD) and other medial or lateral tendon dysfunctions.

DE 20 2004 013 159 U1 discloses an elastic sock-like bandage made from a textile material. At the instep a longitudinal insert made of a highly elastic textile material is attached to the sock-like bandage. Furthermore, a cushion being U-shaped in a horizontal section encloses the rear side of the foot and laterally encloses and protects the Achilles tendon.

US 9,357,804 B2 discloses an ankle gear having a shoe-shaped geometry. The n ankle gear comprises a flexible sleeve which is tensioned by a strap with a Velcro^{®} fastener for encircling the lower leg of the user. Achilles buttresses are interposed between the sleeve and the lower leg of the user. Here, the Achilles buttresses are positioned such that the first Achilles buttress extends vertically along one side of the Achilles tendon and the second Achilles buttress extends vertically along the opposite side of the Achilles tendon. The Achilles buttresses are substantially elliptical and made of a compressible material as a foam or gel-like material. The Achilles buttresses are intended for stimulation the peroneus longus (PL), peroneous brevis (PB) and/or tibialis anterior (TA) muscle spindles.

### OBJECT OF THE INVENTION

It is the object of the present invention to provide an Achilles tendon orthosis which is in particular improved with respect to
- the mechanical interaction with the foot and the Achilles tendon,
- the application of pressure for compressing the foot in particular in the region of the Achilles tendon or intermediate the Achilles tendon or anterior to the mid-portion Achilles tendon,
- the comfort of the person wearing the Achilles tendon orthosis,
- the effort for applying the Achilles tendon,
- the dimensions of the Achilles tendon orthosis,
- the options for adjusting the impact of the Achilles tendon orthosis upon the foot,
- the options for adapting the Achilles tendon orthosis to different persons having differing anatomies,
- the continuous adjustment of the Achilles tendon orthosis and/or the exchange of single parts of the Achilles tendon orthosis for adapting the dimensions and characteristics of the Achilles tendon orthosis and/or
- the costs for manufacturing the Achilles tendon orthosis.

### SOLUTION

According to the present invention, the object of the invention is solved by the features of the independent claim. Additional preferred embodiments according to the invention are to be seen in the dependent claims.

### DESCRIPTION OF THE INVENTION

The present invention relates to an Achilles tendon orthosis for an application to a foot. Here (possibly in contrast to other definitions), the "foot" denotes the part of the human body including the forefoot, the heel, the dorsum of the foot, the ankle joint, the Achilles tendon, the instep and the lower end portion of the lower leg.

The inventive Achilles tendon orthosis is designated for a foot comprising an Achilles tendon having an orientation Y. Furthermore, the outsides of the skin parts covering the Achilles tendon (at locations adjacent to the Achilles tendon) have a distance X. This distance results from the sum of the lateral dimension of the Achilles tendon, the lateral extension of the adjacent skin parts and the lateral extension of the tissue and other parts being arranged between the Achilles tendon and the adjacent skin parts. Finally, the foot to be treated by the inventive Achilles tendon orthosis has an outer contour in a cross section which is taken approximately perpendicular to the orientation Y through the Achilles tendon (preferably in a mid-portion of the Achilles tendon, in the portion of the Achilles tendon where the Achilles tendon has a maximum cross sectional dimension or a cross sectional dimension being larger than 50 %, 40 %, 30 % or 20 % of the maximum cross sectional dimension).

The inventive Achilles tendon orthosis comprises a compression structure. The compression structure extends in a compression structure plane which has an orientation (approximately) perpendicular to the orientation Y. The compression structure serves for generating a compression force when applying the Achilles tendon orthosis to the foot. The inventive Achilles tendon orthosis comprises two compression elements. The compression elements form elongate opposing contact surfaces having an orientation parallel to the orientation Y. The compression elements are designated for contacting the foot in the region of the contact surfaces. The compression elements are supported by the compression structure. Accordingly, the compression forces generated by the compression structure are transferred via the compression elements and their contact surfaces to the foot for providing the pressure generating the healing effect.

According to the invention the Achilles tendon orthosis comprises a biasing mechanism. The biasing mechanism allows a change of the configuration of the Achilles tendon orthosis between two different states, namely a non-biasing state and a biasing state. In the non-biasing state the contact surfaces have a distance which is larger than or equal to the distance X. Accordingly, in the non-biasing state it is possible to apply the Achilles tendon orthosis by moving or sliding the compression elements with a gap or without excess friction to the point of destination (preferably anterior to the mid-portion Achilles tendon and posterior to the end portion of the lower leg and/or to the ankle joint). Instead, in the biasing state the contact surfaces have a distance being smaller than X when no compression counterforces are applied to the contact surface of the compression elements. Accordingly, in the biasing state the distance of the contact surfaces is smaller than X when the Achilles tendon orthosis is not applied to the foot. However, when the Achilles tendon orthosis is applied to the foot and is in its biasing state, compression counterforces generated by the compression structure are effective at the contact surface of the compression elements. These compression forces bias the foot for providing the healing effect. The compression forces might leads to a compression that might lead to a (very small) reduction of the distance X of the skin covering the Achilles tendon and/or the compression counterforce applied by the skin to the compression elements might lead to an elastic deformation of the Achilles tendon orthosis.

According to the invention the Achilles tendon orthosis is dimensioned, configured and designed and in particular individually adapted such that when applying the Achilles tendon orthosis to the foot of the wearer the contact surfaces of the compression elements contact the foot (only or also) at a location anterior to the mid-portion Achilles tendon and posterior to the lower leg and/or ankle joint.

In particular, the inventive Achilles tendon orthosis is laterally supported in a region anterior to the ankle joint with a compression force which is higher than any compression force applied laterally to the ankle joint. Preferably, the inventive Achilles tendon orthosis is not supported in lateral direction at the foot in the region of the ankle joint but only anterior to the same.

Furthermore, preferably the inventive Achilles tendon orthosis does not provide any fixation of the ankle joint and does not bias the ankle joint with any moment providing a resistance against a movement of the ankle joint.

The healing effect of the inventive Achilles tendon orthosis might base on a plurality of reasons. One possible reason is the fact that it has shown that decreased vascularisation has a positive long-term effect on mid-portion Achilles tendinopathy. It is possible that focused controlled continuing bilateral preachillear pressure produces limited blood supply and as a result a decrease of neovascularisation. Consequently, a further decrease of tendon thickness and reduced symptoms and pain is to expect when using the inventive Achilles tendon orthosis. The Achilles tendon orthosis will apply pressure parallel but anterior to the main body of the tendinopathic Achilles tendon. The pressure will not be applied on the tendon itself. For one embodiment the Achilles tendon orthosis is dimensioned such that the Achilles tendon orthosis does not apply pressure medially across the neurovascular bundle of the ankle. One positive effect achieved by the application of the inventive Achilles tendon orthosis might be that the compression destroys the neovessels before they enter into the Achilles tendon. A further formation of neovessels in particular at the Kager's fad pad can be reduced or stopped and existing neovessels can be reduced. It is possible that the compression caused by the inventive Achilles tendon orthosis targets the Kager's fad pad and its neovessels which are formed at the tendinopathic Achilles tendon. For some embodiments the application distally to the Retrocalcanial Bursa is to be avoided. Furthermore, the neurovascular bundle which is lying much deeper should not be affected by the inventive Achilles tendon orthosis. The inventive Achilles tendon orthosis might base on a focused bilateral localised controlled pressure application at the point of pathology. It is e.g. possible that the inventive Achilles tendon orthosis is applied once or twice a day for one to three hours each time wherein the Achilles tendon orthosis is used for six weeks in these intervals.

It is possible that the Achilles tendon orthosis is dimensioned, configured and designed (and in particular individually adapted) such that when applying the Achilles tendon orthosis to a foot the compression structure does not contact the foot along its whole circumference but delimits an intermediate space between the compression structure and the foot. This intermediate space is in particular delimited by pulling elements that bias supporting elements that have an inherent rigidity and serve for biasing the supporting elements with a pulling force. In this case by means of the supporting element the pulling force can be transformed to a compression force which is then applied via the contact surface of the compression elements to the skin of the wearer of the Achilles tendon orthosis.

Within the frame of the invention the compression elements might have any dimensions, contours and designs. For one embodiment the compression elements and/or the contact surfaces of the compression elements have a length in the range of 4 cm to 8 cm, in particular 5 cm to 7 cm. This length corresponds to the length of the portion anterior to the Achilles tendon where it has shown that the application of pressure leads to a healing effect.

The present invention covers embodiments where the position of the compression elements at the compression structure is fixed. For one embodiment of the inventive Achilles tendon orthosis the positions where the compression elements are supported by the compression structure are adjustable. It is possible that the positions are adjustable in circumferential direction of the compression structure, in a direction parallel to the Y-direction and/or an angle of the orientation of the compression elements is adjustable. By an adjustment of the positions it is possible to adapt the Achilles tendon orthosis to the anatomy of the specific wearer. An adjustability can be provided by various measures. To mention only some non-limiting examples the compression structure might be connected to the compression elements by a hook-and-loop-fastener or mushroom-and-loop-fastener for providing the adjustability. It is also possible that the compression elements are adhered to the compression structure after choosing the desired position and/or angle of the compression elements relative to the compression structure. Furthermore, it is possible to mount the compression elements to the compression structure by screwing wherein the compression elements can be screwed to the compression structure at different distinct positions by using different screwing bores or by using elongated holes for providing the desired adjustability.

According to the invention the compression structure comprises supporting elements that have an inherent rigidity. Here, an inherent rigidity of a component as the supporting element means that the component is form stable so that when not applying forces to the component the component has a specific shape. A component having an inherent rigidity might have any elasticity or stiffness lager than zero so that the components might deform under the bias with a force or a torque.

The supporting elements extend preferably in the compression structure plane and (with or without an intermediate space) along the outer contour of the foot. The supporting elements might have any linear or curved longitudinal extension and might e.g. be formed by a ring segment, a straight or curved rod or beam and the like. The supporting elements support the compression elements. Due to the compression counterforces acting upon the contact surfaces of the compression elements the supporting elements are biased by a bending moment. The bending axis of the bending moment has an orientation parallel to the Achilles tendon (so to the direction Y). Accordingly, the supporting elements are used as a kind of beam which is bent in the case that the supporting elements have an elasticity. This inventive embodiment has the advantage that by the choice of the dimensions of the bending beam formed by the supporting element and by the choice of the location of the application of the compression counterforce to the bending beam and of the location of the application of the connecting forces of other connecting elements linked to the supporting elements it is possible to design the compression force generation characteristics of the inventive Achilles tendon orthosis. The supporting elements might also be embodied as compression structure parts as these will be described below.

The supporting element might have any design and shape. For a particular proposal of the invention at least one of the supporting elements is H-shaped, V-shaped or U-shaped. Here, the side legs of the H or U might have the same or different lengths. The base leg and the side legs of the H or U might be linear or have any curvature or might be adapted to the outer contour of the foot. Side legs might protrude from a base leg of the H or U under an angle of 90° or different angles between 70° and 120°. The side legs of the H or U might have a parallel orientation or might be slightly inclined. In particular the base leg of the H or U serves as a support for the compression element. In particular the base leg of the H or U has a length equalling or exceeding the length of the compression elements or the contact surfaces of the compression elements. The two side legs of the U, V or H might have a distance which is larger than the outer diameter of the convex curvature of the foot at the ankle joint. Accordingly, when applying the Achilles tendon orthosis to the foot the side legs of the U, V or H are able to pass above and below the convex curvature of the foot at the ankle joint which leads to an increased comfort of the wearer. In this case connecting elements might each comprise two connecting element parts or branches that are linked to the end portions of the side legs of the U, V or H. The supporting elements might have a constant or varying stiffness and/or cross sectional area in their longitudinal direction.

For one embodiment of an inventive Achilles tendon orthosis at least one portion of the compression structure (in particular a supporting element) having an inherent rigidity comprises a local weakening. In the case that the supporting elements are H-, V- or U-shaped the base leg and/or at least one side leg might comprise the local weakening. The local weakening allows an elastic deflection of the supporting element under the bias of the applied forces. If the supporting element is biased by pulling forces by connecting elements and/or by the compression counterforce the resulting bending moment leads to a pivoting movement of the sections of the supporting element on both sides from the local weakening under an elastic deformation of the weakening. Accordingly, the supporting element might automatically adapt to the outer contour of the foot. The local weakening might be formed by a groove extending in lateral direction of the supporting element, by a number of bores or any other type of recess.

It is possible that the Achilles tendon orthosis consists of the compression structure and the compression elements whereas it is also possible that the Achilles tendon orthosis comprises additional components. For one embodiment the Achilles tendon orthosis comprises a cover. The cover covers, supports and/or embeds the compression structure and/or the compression element. The cover might be made of any weak or stiff material, in particular a tissue. The cover might also form a kind of sleeve or sock. The cover might extend around a part or the whole circumference of the foot.

For another proposal of the invention the Achilles tendon orthosis comprises a loop-shaped pulling stirrup. The loop-shaped pulling stirrup connects one supporting element to the other supporting element. The pulling stirrup serves for fixing the Achilles tendon orthosis against an undesired movement in upper direction away from the ankle joint. This is provided by passing the pulling stirrup from one supporting element around the bottom side of the foot to the other supporting element.

The invention covers embodiments wherein the compression elements are fixed to the compression structure. However, the invention also proposes an Achilles tendon orthosis wherein the compression elements are supported by the compression structure (in particular the supporting elements) by a guidance. The guidance provides at least one degree of freedom for allowing a relative movement between the compression elements and the compression structure. This degree of freedom might e.g. be a translational degree of freedom or a rotational degree of freedom. The provided degree of freedom might be used for adapting the position of the compression elements relative to the compression structure when applying the Achilles tendon orthosis to the foot in order to guarantee that the compression elements contact the foot at the desired contact area. Alternatively or cumulatively it is possible that the degree of freedom provided by the guidance allows a compensating relative movement between the compression elements and the compression structure when walking or running with the Achilles tendon orthosis applied to the foot. The degree of freedom of the guidance might in particular compensate a pivoting movement of the ankle joint which might be accompanied by a relative movement of the Achilles tendon orthosis and the desired contact area between the compression elements and the foot on the one hand and the other contact areas of the Achilles tendon orthosis with the foot.

The invention covers embodiments of the Achilles tendon orthosis wherein the created compression force is defined by the design and the dimensions of the Achilles tendon orthosis. For one embodiment of the invention the Achilles tendon orthosis comprises a manual actuation element. By means of a manual actuation of the manual actuation element it is possible to adjust the compression force, i. e. to increase or decrease the compression force. Accordingly, by the manual actuation of the manual actuation element it is possible to specifically adapt the created compression force and so the healing effect to the specific application. Here, within the frame of the invention any manual actuation element and related mechanism for changing the dimensions of the Achilles tendon orthosis and for adjusting the compression force can be used. To mention only some non-limiting example the manual actuation element might actuate a tensioning device, e. g. by use of a manual actuation element being embodied as a manual actuation wheel. For the given example a manual rotation of the actuation wheel winds a pulling means for increasing a pulling or tensioning force. It is e. g. also possible that the manual actuation element actuates a linear ratchet mechanism that is used for tensioning a pulling element on the basis of a linear motion instead of a winding motion. The adjustment of the compression force by the manual actuation of the manual actuation element might be induced in two or more steps or also in a continuous fashion.

The present invention in particular proposes two different embodiments of the Achilles tendon orthosis:
a) For a first embodiment the compression structure forms a closed ring. The closed ring extends in the compression structure plane. When applying the Achilles tendon orthosis to the wearer the foot extends through the closed ring. The invention covers both embodiments wherein the ring has a circular shape as well as a ring structure having a non-circular shape, in particular with ring parts having an inherent rigidity and being partially linear or having any curved shape.
   In this case the ring comprises two supporting elements having an inherent rigidity. The supporting elements each support one of the compression elements at a location between the end portions of the supporting element. One end portion of a first supporting element is connected by a first connecting element to an end portion of the second supporting element. The other end portion of the first supporting element is connected by a second connecting element to the other end portion of the second supporting element. Preferably, the connecting elements have no inherent rigidity and serve only for applying a pulling force but no bending moment to the supporting elements. By the two connecting elements the supporting elements are each biased in their end portions with a pulling force.
   The absolute value of the compression force applied by the contact area of the foot equals the sum of the two pulling forces applied by the two connecting elements to the supporting element. In the case that the supporting element is not only supported via the compression element at the foot the sum of the aforementioned forces might divide to a compression force being transferred by the compression element and a force component applied to another part of the foot. In this case the biasing mechanism which allows a non-biasing state and a biasing state is formed by an adjustable effective length of at least one connecting element linked to the first and second supporting element.
   As a connecting element any component suitable for applying a pulling force upon the supporting element and connecting the two supporting elements might be used. For one embodiment at least one connecting element is formed by a wire, thread, strip or filament. The wire, thread, strip or filament is linked in the end regions and/or in at least one middle section to the supporting elements. The wire, thread, strip or filament is biased by a pulling force that is transferred to the supporting elements. Furthermore, the connecting element comprises a ratchet mechanism. By a manipulation of the ratchet mechanism it is possible to adjust the effective length of the wire, thread, strip or filament. As a ratchet mechanism a device might be used which includes a spool upon which with a rotation of a manipulation element the wire, thread, strip or filament is wound. A ratchet allows a movement of the spool for winding the wire, thread, strip or filament but blocks a rotation of the spool in opposite direction for unwinding the wire, thread, strip or filament. By a manually actuated release element or release button it is possible to unlock the ratchet for allowing an unwinding rotation of the spool.
   Ratchet mechanisms of this type are e.g. distributed under the trademark "BOA" (registered trademark) or other similar embodiments. By a manipulation of the ratchet mechanism, here a manually caused rotation of the spool, it is possible to tighten the Achilles tendon orthosis at the foot by reducing the effective length of the wire, thread, strip or filament.
   For another embodiment at least one of the connecting elements comprises at least one strip of any dimension, e.g. made of a fabric. The strip might have a hook-and-loop-fastener or mushroom-and-loop-fastener for adjusting the effective length. It is e.g. possible that a connecting element comprises two strips. One end region of each of the strips is linked to an associated supporting element. The other end regions of the two strips are connected to each other by a hook-and-loop-fastener wherein the hook-and-loop-fastener allows an individual adaptation of the effective length resulting from the sum of the effective lengths of the two strips and allows to adjust the pulling force of the connecting element built in this way.
   For a preferred embodiment of the Achilles tendon orthosis comprising connecting elements embodied as a wire, thread or filament, the wire, thread or filament forms a tackle. The use of a tackle is advantageous because for small manipulation forces biasing the ratchet mechanism it is possible to produce large pulling forces in the connecting element.
b) For a second embodiment of the invention the compression structure is bracket-shaped, C-shaped, V-shaped or U-shaped. Accordingly, the compression structure is not a closed ring but comprises an opening. The rear side of the foot with the Achilles tendon can be introduced from the front side into the opening of the bracket, C, V or U so that the compression structure accommodates the posterior part of the foot with the Achilles tendon between side legs of the bracket, C, V or U. For this embodiment the compression structure and/or the compression elements are/is elastic. Due to the elasticity it is possible that in the biasing state of the Achilles tendon orthosis the compression elements are pressed by an elastic tension of the compression structure and/or the compression elements against the foot of the wearer at a position anterior to the mid-portion of the Achilles tendon and posterior the end portion of the lower leg and/or the ankle joint. In the non-biasing state of the Achilles tendon orthosis the elastic bias of the compression structure and/or the compression elements is higher than in the biasing state of the Achilles tendon orthosis. Said in different words: in the non-biasing state the elasticity of the compression structure and/or the compression elements tends to press the contact surfaces of the compression elements towards the contact area of the foot located anterior to the mid-portion Achilles tendon. In this case the compression elements are supported at the end portions of the compression structure(so at the end portions of the side legs of the bracket, C, V or U). For the length, angles and shapes of the legs of the bracket, C, V, U the above stated for the different legs of the H, V or U formed by the supporting elements also applies.
   In this case it is possible that the compression structure comprises only one single bracket, C-shaped rod or beam, V-shaped rod or beam or U-shaped rod or beam which holds the compression elements in its end region. For another embodiment of the invention the compression structure comprises two approximately parallel compression structure branches which are connected in their end regions to the compression elements. To mention only one non-limiting example it is possible that for a projection of the Achilles tendon orthosis into one plane or for a developed view the Achilles tendon orthosis forms (in a rough approximation) a rectangle wherein two opposing sides are formed by the compression structure branches whereas the other two opposing sides are formed by the compression elements. Preferably, the length of the compression elements is in the range of 4-8 cm (in particular 5 cm to 7 cm) whereas the length of the compression structure branches might be larger than that of the compression elements.
   It is e. g. possible that the compression structure is made as one single integral part or made of two single integral compression structure branches. Furthermore it is possible that the compression structure comprises a definite shape which can only be changed due to an elastic deflection of the compression structure. However, the invention also proposes that the Achilles tendon orthosis comprises an adjusting mechanism. Upon manipulation of the adjusting mechanism it is possible to adjust at least one dimension and/or the shape of the compression structure. To mention only one non-limiting example the compression structure or the compression structure branches might comprise two compression structure parts that are linked to each other by a sliding guidance or by a pivot joint, the pivot joint having a pivot axis having an orientation parallel to the orientation Y of the Achilles tendon. Caused by the relative movement of the two compression structure parts due to a manipulation the dimension and/or shape of the compression structure can be adjusted. Here, the adjustment can be used for adapting the Achilles tendon orthosis to different anatomies of the wearer and/or to adjust the compression forces applied by the Achilles tendon orthosis to the contact area of the foot.
   Furthermore the invention proposes that the compression structure or the compression structure branches comprises or comprise two compression structure parts being connected to each other in neighbouring end regions by a joint. The joint defines a pivot axis having an orientation parallel to the Y-direction. In the other end regions the compression structure parts are connected to an associated compression element. For a first embodiment a ratchet mechanism allows a pivoting movement of the compression structure parts about the pivot axis in closing direction but blocks a pivoting movement of the compression structure parts about the pivot axis in opening direction. Also in this case there can be a manipulation button for releasing the ratchet mechanism. By a ratcheting pivoting movement of the compression structure parts towards each other it is possible to adapt the Achilles tendon orthosis to a wearer having a foot of smaller dimensions and/or to increase the compression force biasing the foot of the wearer. For another embodiment the two compression structure parts are linked to each other by a spring biasing the two compression structure parts for pivoting about the pivot axis of the joint in closing direction.

Advantageous developments of the invention result from the claims, the description and the drawings. The advantages of features and of combinations of a plurality of features mentioned at the beginning of the description only serve as examples and may be used alternatively or cumulatively without the necessity of embodiments according to the invention having to obtain these advantages. Without changing the scope of protection as defined by the enclosed claims, the following applies with respect to the disclosure of the original application and the patent: further features may be taken from the drawings, in particular from the illustrated designs and the dimensions of a plurality of components with respect to one another as well as from their relative arrangement and their operative connection. The combination of features of different embodiments of the invention or of features of different claims independent of the chosen references of the claims is also possible, and it is motivated herewith. This also relates to features which are illustrated in separate drawings, or which are mentioned when describing them. These features may also be combined with features of different claims. Furthermore, it is possible that further embodiments of the invention do not have the features mentioned in the claims.

The number of the features mentioned in the claims and in the description is to be understood to cover this exact number and a greater number than the mentioned number without having to explicitly use the adverb "at least". For example, if a spring or a connecting element is mentioned, this is to be understood such that there is exactly one spring or one connecting element or there are two springs or two connecting elements or more springs or more connecting elements. Additional features may be added to these features, or these features may be the only features of the respective product.

The reference signs contained in the claims are not limiting the extent of the matter protected by the claims. Their sole function is to make the claims easier to understand.

### BRIEF DESCRIPTION OF THE DRAWINGS

In the following, the invention is further explained and described with respect to preferred exemplary embodiments illustrated in the drawings.
- **Fig. 1**: shows a lateral X-ray of a foot in the region of the ankle joint, the lower end region of the lower leg and the Achilles tendon.
- **Fig. 2**: shows the X-ray of Fig. 1 wherein the contact area is marked where compression forces are applied to the foot by the inventive Achilles tendon orthosis.
- **Fig. 3**: shows a schematic sectional view of an Achilles tendon orthosis applied to a foot with a sectional plane perpendicular to a longitudinal axis Y of the Achilles tendon.
- **Fig. 4**: in a schematic sectional view corresponding to Fig. 3 shows a free body diagram of supporting elements of the Achilles tendon orthosis of Fig. 3 with the applied compression counterforces and the pulling forces applied by the connecting elements.
- **Figs. 5 to 9**: show different views of a supporting element, namely Fig. 5 a lateral outer view, Fig. 6 a front view, Fig. 7 a rear view, Fig. 8 a lateral inner view of one single left supporting element whereas Fig. 9 shows a set of supporting elements in a three-dimensional view from the top.
- **Figs. 10 to 11**: show three-dimensional views of supporting elements as shown in Figs. 7 to 9 when applied to a foot (connecting elements not shown).
- **Fig. 12**: shows an Achilles tendon orthosis applied to a foot, the Achilles tendon orthosis comprising supporting elements as shown in Figs. 5 to 11 being connected to each other by a wire where it is possible to change the effective length of the wire and increase the compression force by manipulation of a ratchet mechanism.
- **Fig. 13**: shows an Achilles tendon orthosis applied to a foot, the Achilles tendon orthosis comprising supporting elements as shown in Figs. 5 to 11 being connected to each other by strips having hook-and-loop-fasteners.
- **Figs. 14 and 15**: show an Achilles tendon orthosis applied to a foot in a three-dimensional view wherein the supporting elements are embedded or covered by a cover.
- **Fig. 16**: shows an Achilles tendon orthosis according to Figs. 14 and 15 in a three-dimensional view with an additional loop-shaped pulling stirrup which connects the two supporting elements.
- **Figs. 17 and 18**: show another embodiment of an Achilles tendon orthosis where Fig. 17 is a three-dimensional view and Fig. 18 is a top view.
- **Figs. 19 and 20**: in three-dimensional views show the Achilles tendon orthosis of Figs. 17 and 18 applied to a foot.
- **Figs. 21 and 22**: show another embodiment of an Achilles tendon orthosis where Fig. 21 is a three-dimensional view and Fig. 22 is a top view.
- **Figs. 23 and 24**: in three-dimensional views show the Achilles tendon orthosis of Figs. 21 and 22 applied to a foot.
- **Figs. 25 and 26**: in three-dimensional views show another embodiment of an Achilles tendon orthosis where Fig. 25 is a top view from the front and Fig. 26 is a top view from the back.
- **Fig. 27**: is a three-dimensional view showing supporting elements of an Achilles tendon orthosis of Figs. 25 and 26 with compression elements at the designated positions when applying the Achilles tendon orthosis to a foot.

### DESCRIPTION OF THE DRAWINGS

In the following description of the drawings components of different embodiments having the same or similar designs, dimensions and/or functions are partly denoted with one and the same reference numeral. In the case that in one embodiment one and the same component or part of a component is present a number of times, these components or parts of the components are denoted with one and the same reference numeral but distinguished from each other by additional letters a, b, ... However, in this case reference to these components or part of the components can also be made without use of the additional letter a, b, ...

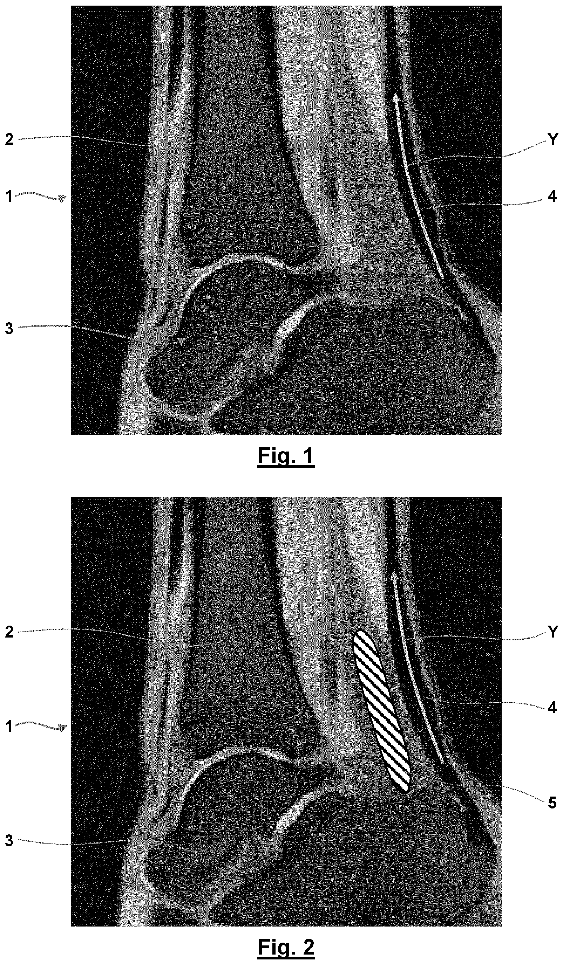

**Fig. 1** shows a lateral X-ray of a foot 1 in the region of an end portion of a lower leg 2, an ankle joint 3 and an Achilles tendon 4 having a (straight or slightly curved) longitudinal axis Y.

**Fig. 2** shows the contact area 5. The contact area 5 is preferably located anterior to the mid-portion Achilles tendon 4 and posterior to the end portion of the lower leg 2 and/or the ankle joint 3. By the inventive Achilles tendon orthosis 6 compression forces are applied to contact areas 5 located on opposite lateral sides of the foot 1. The contact area 5 has a longitudinal extension of 4 to 8 cm (in particular 5 to 7 cm) in a direction parallel to the longitudinal direction Y of the Achilles tendon 4. The contact area 5 is elongated and has a width preferably in the range of 0,5 to 2 cm, in particular 0,8 to 1,5 cm. The contact area 5 is arranged between the Achilles tendon 4 and the ankle joint 3 or the lower leg 2 in the lateral X-ray of Fig. 2.

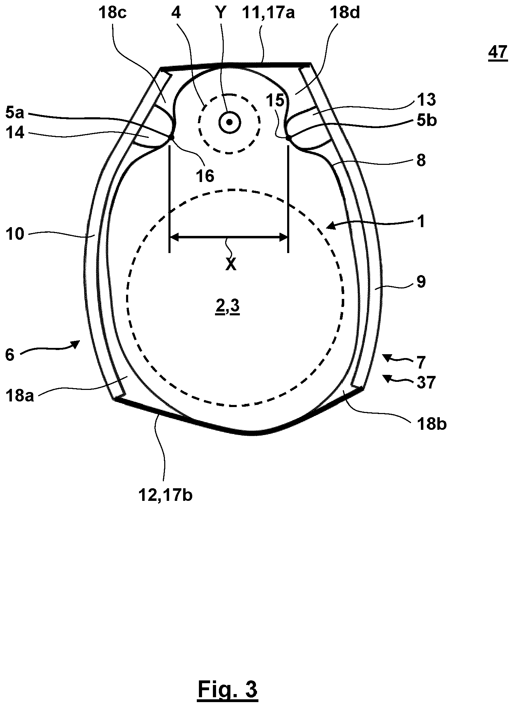

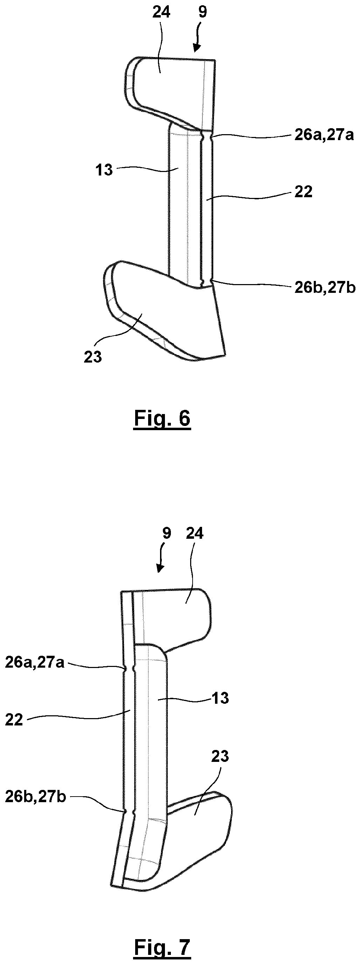

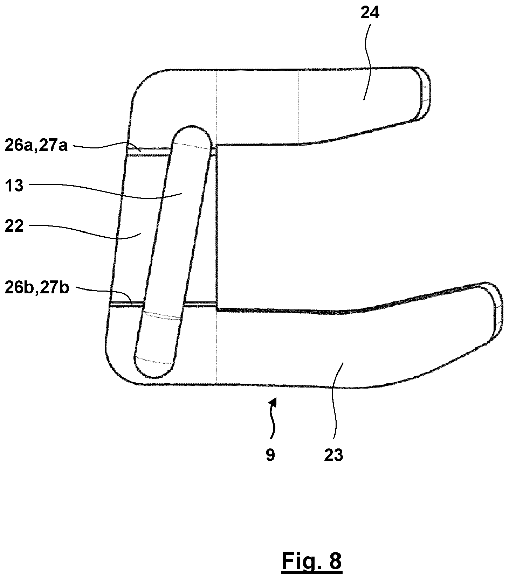

**Figs. 3 to 16** show a first design variant of the Achilles tendon orthosis 6 in different embodiments.

According to a schematic cross section taken perpendicular to a longitudinal axis Y of the Achilles tendon 4 or a top view of Fig. 3 the Achilles tendon orthosis 6 forms a ring 7 extending around the circumference of an outer contour 8 of the foot 1 in the region of the ankle joint 3 or the Achilles tendon 4.

The ring 7 comprises opposite supporting elements 9, 10. The supporting elements have an inherent rigidity. In the end regions the supporting elements 9, 10 are connected to each other by connecting elements 11, 12. Preferably, the connecting elements 11, 12 have no inherent rigidity or a bending elasticity being by a factor of at least 10 smaller than the bending elasticity of the supporting elements 9, 10. When seen in circumferential direction the closed ring 7 is formed by the supporting element 9, the connecting element 11, the supporting element 10 and the connecting element 12 in the aforementioned order.

The supporting elements 9, 10 each carry or hold an associated compression element 13, 14 on the side facing towards the outer contour 8 of the foot 1. The compression elements 13, 14 have an elongate shape with a longitudinal direction perpendicular to the drawing plane of Fig. 3. The compression elements 13, 14 are elastic, comprise a cushion or a kind of pillow for providing soft contact surfaces 15, 16 which apply the compression forces to the contact areas 5a, 5b of the foot 1. It is possible that the supporting elements 9, 10 are deflected or bent by the acting forces. However, it is also possible that the supporting elements 9, 10 have a bending stiffness such that they are not deform to a considerable extend under the effective forces.

The connecting elements 11, 12 might e.g. be formed by at least one wire, thread, strip or filament 17. The wire, thread, strip or filament 17 is biased by a pulling force tending to pull the associated ends of the supporting elements 9, 10 towards each other.

The compression elements 13, 14 can be located at any position along the supporting element 9, 10. Preferably, the compression element 13, 14 is arranged adjacent to the rear end of the supporting element, e.g. in the region of 10 % to 30 % of the extension of the supporting element 9, 10 when seen in circumferential direction around the outer contour 8 starting from the rear end.

It is possible that the supporting elements 9, 10 have a longitudinal extension such that they protrude in anterior and/or posterior direction and such that the connecting elements 11, 12 are straight and do not contact the outer contour 8 of the foot or form a tangent to the outer contour 8. However, it is also possible that the connecting elements 11, 12 on the front side of the outer contour 8 and/or on the rear side of the outer contour 8 contact and follow the outer contour 8 of the foot as shown in Fig. 3.

Furthermore, it is possible that at least one intermediate space 18 is formed between the components of the Achilles tendon orthosis 6 and the outer contour 8 of the foot 1. For the embodiment shown in Fig. 3 there is an intermediate space 18a being delimited by a right part of the outer contour 8, the compression element 14, the front part of the supporting element 10 and the connecting element 12. The corresponding applies to an intermediate space 18b arranged on the left side. Furthermore, an intermediate space 18c is formed on the right side and is delimited by the rear end of the supporting element 10, the compression element 14, the rear right part of the outer contour 8 and the connecting element 11. The corresponding applies for an intermediate space 18d.

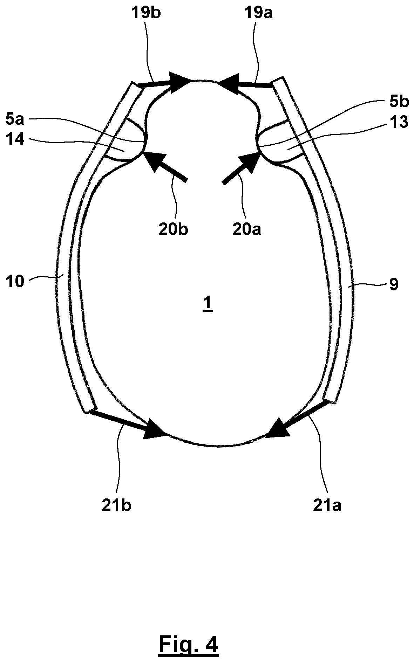

Fig. 4 schematically shows a free body diagram of the supporting elements 9, 10. Pulling forces 19a, 19b of the connecting element 11 bias the rear ends of the supporting elements 9, 10. The compression elements 13, 14 are biased by compression counterforces 20a, 20b applied by the foot 1 in the contact areas 5 to the compression elements 13, 14. The compression counterforces 20a, 20b are the reactive forces opposing the compression forces (here not shown) applied to the contact areas (5a, 5b). Furthermore, pulling forces 21a, 21b bias the front ends of the supporting elements 9, 10. The pulling forces 21 are applied by the connecting element 12to the supporting elements 9, 10.

For a simplified consideration (differing from the direction of the forces 19, 20, 21 in Fig. 4) it is assumed that the forces 19, 20, 21 have a parallel orientation. Under this assumption the absolute value of the compression counterforces 20 equals the sum of the absolute values of the pulling force 19 and the pulling force 21 being applied to the supporting element 9, 10. Here, the pulling forces 19, 21 have differing absolute values according to the differing lever arms with respect to the support of the compression elements 13, 14 at the foot 1. When considering the non-parallel orientations of the forces 19, 20, 21 as shown in Fig. 4 the compression counterforces 20 result from the sum of the vectors of the pulling force 19 and the pulling force 21. The differing absolute values and orientations of the pulling force 19 and the pulling force 21 result from the mechanics and from the chosen shapes and lengths of the supporting elements 9, 10.

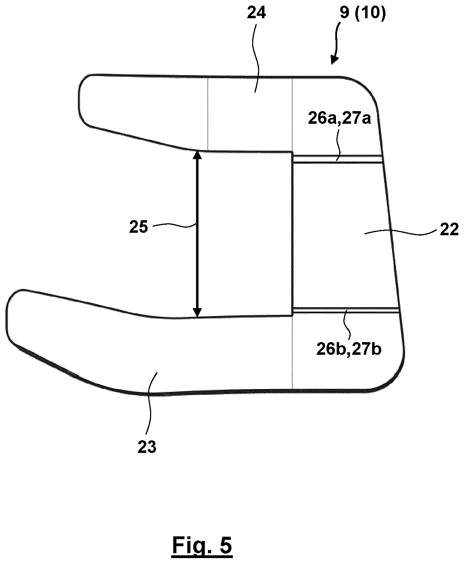

It is generally possible that the supporting elements 9, 10 consist of a (straight or curved) bar, beam or rod. However, Figs. 5 to 8 show supporting elements 9, 10 being U-shaped and comprising a base leg 22 and two side legs 23, 24. The base leg 22 has an orientation parallel to the longitudinal direction Y of the Achilles tendon 4 whereas the side legs 23, 24 extend in circumferential direction of the outer contour 8 of the foot 1. For the shown example the side legs 23, 24 have different lengths where the lower leg is longer than the upper leg. The base leg 22 and the side legs 23, 24 are here formed by one integral part. The extension of the base leg 22 is preferably 4 to 8 cm or 3 to 7 cm or such that the side legs 23, 24 have a distance 25 being larger than the outer convex curvature 38 of the skin of the foot 1 resulting from the ankle joint 3. Accordingly, it is possible to accommodate the convex curvature 38 of the ankle joint 3 between the two side legs 23, 24. As an optional feature the supporting elements 9, 10 might comprise at least one weakening 26 which is here formed by a groove 27. For the shown embodiment the grooves 27 are provided at the points of transition of the base leg 22 to the side legs 23, 24. The grooves 27 extend in circumferential direction of the outer contour 8, so parallel to the side legs 23, 24. The side legs 23, 24 are curved in longitudinal direction in the compression structure plane 47 in order to follow the outer contour 8 of the foot 1 with or without establishing a gap or intermediate space 18a, 18b. The supporting elements 9, 10 have a plate-like design.

The compression element 13, 14 is attached to the base leg 22 of the supporting element 9, 10 on the side facing towards the foot 1. It is possible that the longitudinal position of the fixation of the compression element 13, 14 at the base leg 22 and its orientation relative to the orientation of the base leg 22 is adjustable.

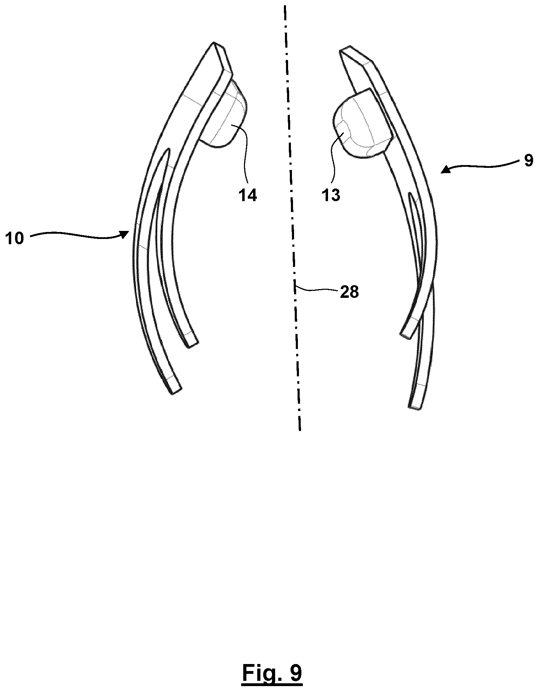

It is possible that the two supporting elements 9, 10 have the same or different shapes. Preferably, the shapes of the supporting elements 9, 10 are mirrored with respect to a plane 28 extending perpendicular to the drawing plane 28 of Fig. 3 and in the middle between the two supporting elements 9, 10 (cp. Fig. 9).

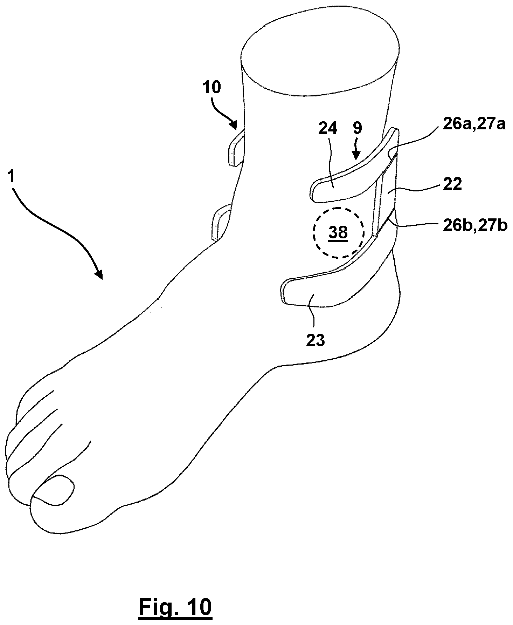

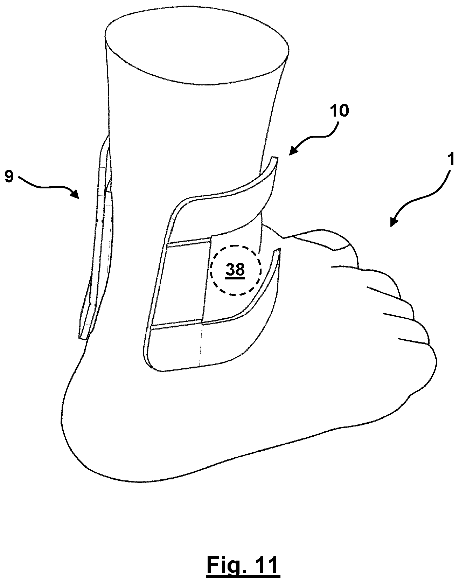

Figs. 10 and 11 show the supporting elements 9, 10 when applied to a foot 1.

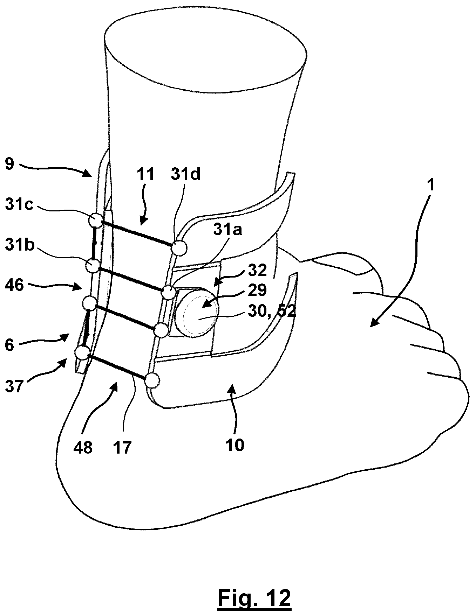

Fig. 12 shows one embodiment of an Achilles tendon orthosis 6 comprising the supporting elements 9, 10 as described before. Here, the connecting element 11 is tightened by a ratchet mechanism 29. The ratchet mechanism 29 comprises a spool that can be rotated by a manual actuation element 52 here embodied as a manual actuation wheel 30 so that two wire parts leading to the spool are simultaneously wound onto the spool leading to a tightening and reduction of the effective length of the wire 17. A ratchet mechanism 29 of this type is e.g. distributed under the trademark "BOA" (registered trademark). However, there are also a lot of different ratchet mechanisms available on the market and suitable for use in the inventive Achilles tendon orthosis 6. The ratchet mechanism 29 allows a rotation of the actuation wheel 30 with the associated spool only in one direction leading to a tightening of the wire 17. Generally the rotation in the opposite direction is blocked by the ratchet mechanism 29. However, the ratchet mechanism 29 can be released so that a rotation in opposite direction is allowed by a manipulation of a release button (no shown).

For the shown embodiment two wire parts of the wire 17 extend from the ratchet mechanism 29. The wire parts are each passed through an eye 31a of the supporting element 10, bridge the distance between the supporting elements 10 and 9, are passed through adjacent eyes 31b, 31c of the supporting element 9, then bridge the distance between the supporting elements 9, 10 and end in an eye 31d of the supporting element 10. In this way a kind of tackle 32 is formed.

Here, the connecting element 12 linked to the front ends of the supporting elements 9, 10 is not shown. For the connecting element 12 any adjustable or non-adjustable connecting element 12 (in particular at least one wire, thread, strip or filament 17) can be used.

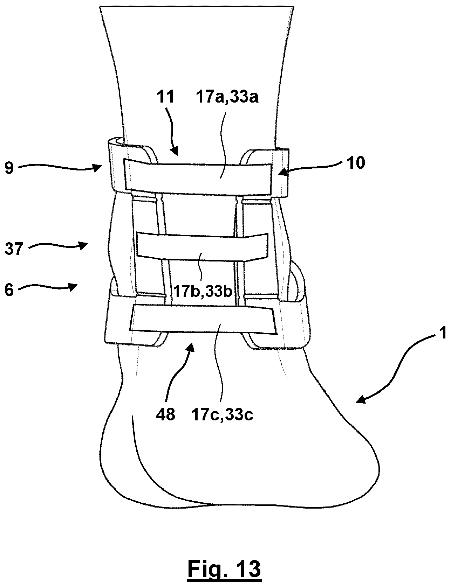

For the embodiment shown in Fig. 13 instead of a wire 17 tightened by a ratchet mechanism 29 strips 17 comprising hook-and-loop-fasteners 33 are used. The hook-and-loop-fasteners 33 form the connecting element 11 and allow an adjustment of the effective length of the strips 17 for applying the required pulling forces 19a, 19b.

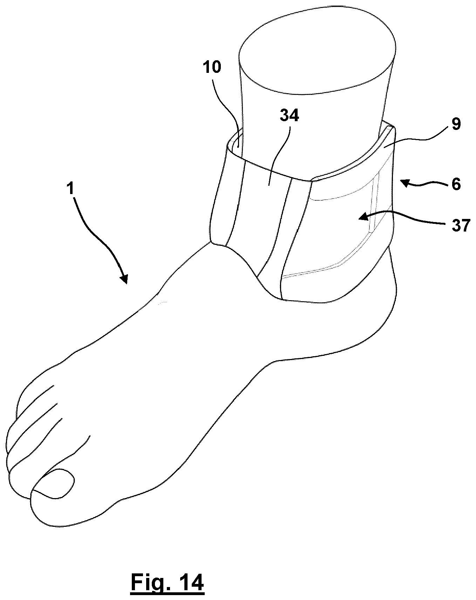

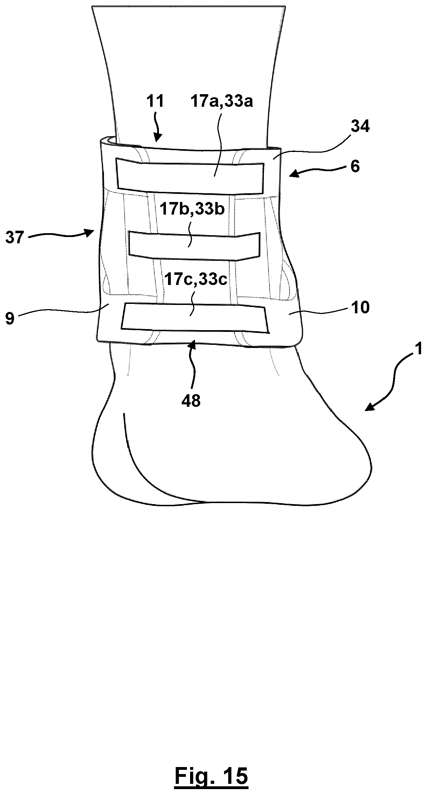

Fig. 14 shows an embodiment wherein the supporting elements 9, 10 are embedded in or attached to a cover 34. In Fig. 14 the supporting elements 9, 10 are shown in thin dotted lines due to the fact that these are covered by the cover 34 and not visible. The cover 34 might extend along the whole circumference of the contour 8 or only in at least one part of the circumference. For the given example the cover 34 also forms the connecting element 12. As shown in Fig. 15 on the rear side of the Achilles tendon orthosis 6 the cover 34 might carry or be connected to a connecting element 11, in particular the ratchet mechanism 32 and the wire 17 or (as shown in Fig. 15) two strips 17 comprising the hook-and-loop-fasteners 33.

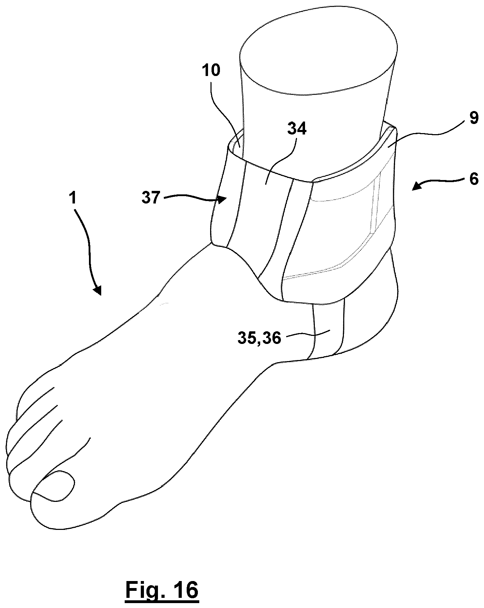

For the embodiments shown in Figs. 12 to 15 the Achilles tendon orthosis 6 has a sleeve- or tubelike shape. The longitudinal axis of the tube or sleeve has an orientation parallel to the orientation of the lower leg 2 and to the longitudinal axis Y of the Achilles tendon 4. The Achilles tendon orthosis 6 is located at and/or above the instep of the foot 1. For the embodiment shown in Fig. 16 the Achilles tendon orthosis 6 comprises an additional strip 35 connecting the supporting elements 9, 10 or the cover 34 and being passed around the sole of the foot 1. Accordingly, the strip 35 forms a kind of loop-shaped pulling stirrup 36. A movement of the ring part of the Achilles tendon orthosis 6 in lower direction is not possible due to the extending cross sectional diameter of the foot 1 in the region of the instep. A movement of the ring-shaped part of the Achilles tendon orthosis 6 in upper direction is avoided by the strip 35 or pulling stirrup 36.

For the embodiments of the first variant the Achilles tendon orthosis 6 comprises a ring-shaped part wherein in the ring-shaped part alternatingly supporting elements 9, 10 having an inherent rigidity are followed in circumferential direction by connecting elements 11, 12 having no or a smaller inherent rigidity. For applying the Achilles tendon orthosis 6 the ring-shaped part is slid from the front over the foot 1 until its final destination as shown in figures 10, 12, 13, 14, 15, 16. However, it is also possible to open at least one of the connection elements 11, 12 and so the ring 7 for applying the Achilles tendon orthosis 6 without the need to slide the Achilles tendon orthosis 6 from the toes over the foot 1.

The ring 7 forms a compression structure 37 which supports the compression elements 13, 14 and is tightened by increasing the pulling forces in the connecting elements 11, 12. The tightening of the compression structure 37 generates the pulling forces 19, 21 which again result in the compression forces being applied to the foot 1.

For a second variant of the invention the compression elements 13, 14 are supported at a compression structure 37 which is not ring-shaped but (in a first approximation) comprises the shape of a bracket, C, V or U 39when seen in a top view or along the orientation Y in the applied state of the Achilles tendon orthosis 6.

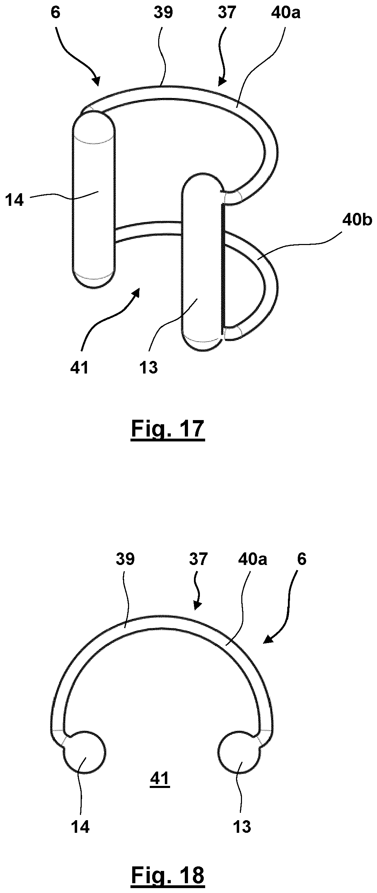

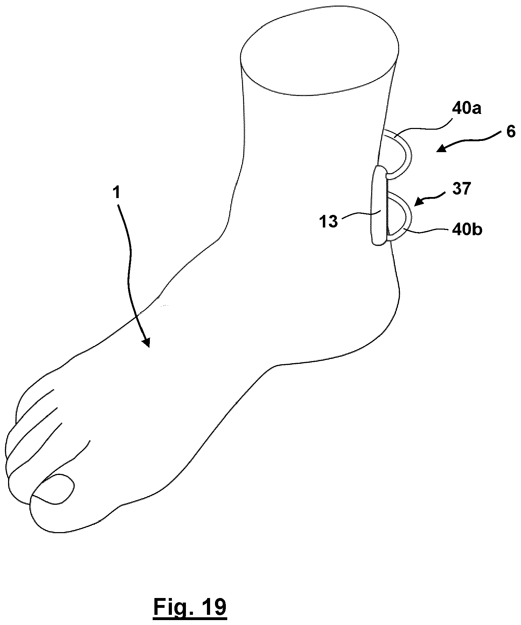

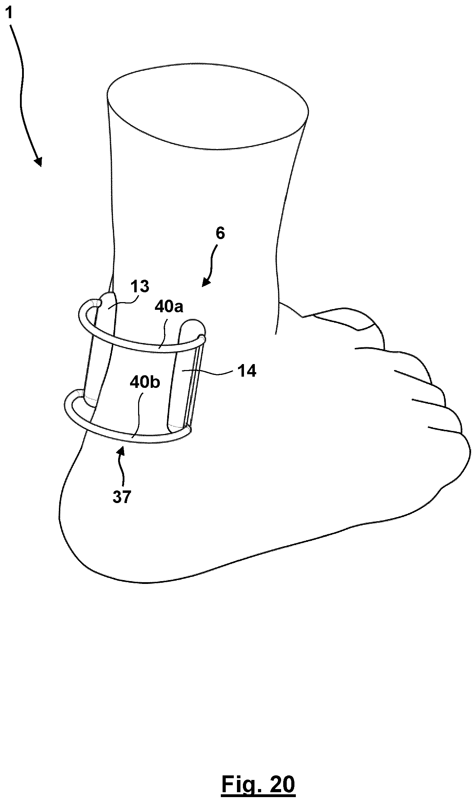

Here, the compression structure 37 consists only of one single supporting element 9 which holds the compression elements 13, 14 in its end, so in the ends of the bracket, C, V or U 39. The Achilles tendon orthosis 6 of the second variant forms a kind of clamp. For the shown embodiment the compression structure 37 is approximately semi-circular wherein the ends of the compression structure 37 are slightly bent in inner direction at the transitions to the compression elements 13, 14. As can be seen in **Fig. 17** for the shown embodiments the compression structure 37, so the supporting element 9 comprises two parallel compression structure branches 40a, 40b. The two compression structure branches 40a, 40b are in their end regions connected to the upper and lower ends of the compression elements 13, 14. **Figs. 19****,** **20** show the Achilles tendon orthosis 6 of **Figs. 17 and 18** being applied to a foot. Due to the design of the compression structure 37 as a bracket C, V or U 39 the compression structure 37 and so the Achilles tendon orthosis 6 has an opening 41. Accordingly, for this embodiment it is possible to introduce the foot with its posterior rear side in the region of the Achilles tendon 4 into the opening 41 and so into the Achilles tendon orthosis 6. The compression structure 37 and/or the compression elements 13, 14 are manually elastically biased such that the distance of the compression elements 13, 14 increases. The elastic bias of the compression structure 37 and/or the compression elements 13, 14 causes the compression force being applied to the contact areas 5a, 5b of the foot 1. The compression counterforces 20a, 20b cause a bending moment being effective about a bending axis which has an orientation parallel to the longitudinal axis of the compression elements 13, 14 (and so parallel to the orientation Y of the Achilles tendon 4). The compression structure branches 40a, 40b are preferably formed by metallic curved rods or a metallic wire having a circular cross section and a diameter in the range of 2.0 to 3.0 mm whereas also a non-circular cross section (in particular with a cross sectional area corresponding to the cross sectional area of a circular cross section with a diameter of 2.0 to 3.0 mm) can be used.

Differing from the embodiments shown in Figs. 18-20 it is possible that the Achilles tendon orthosis 6 additionally comprises a strip which is connected in its end regions to the compression elements 13, 14 and which is passed around the instep of the foot 1. This strip might additionally secure the compression elements 13, 14 at their points of destination. The strip might have an adjustable effective length.

Furthermore, the Achilles tendon orthosis of Figs. 17 to 20 might additionally comprise a strip 35 forming a pulling stirrup 36.

For another embodiment of this variant the compression structure 37, here each of the compression structure branches 40, comprises two compression structure parts 42a, 42b. For the shown embodiment the compression structure parts 42a, 42b are quarter-circular and connected to each other at their adjacent ends by a pivot joint 43. The pivot axis of the pivot joint 43 has an orientation parallel to the longitudinal direction of the compression elements 13, 14 and/or of the orientation Y of the Achilles tendon 4.

It is possible that the compression structure parts 42, 43 are biased by a spring in closing direction so that the compression elements 13, 14 held in the other end regions of the compression structure parts 42a, 42b are biased towards each other for applying the compression force. In this case the elasticity might only be provided by the spring or might be provided both by the spring and the bending elasticity of the compression structure parts 42a, 42b.

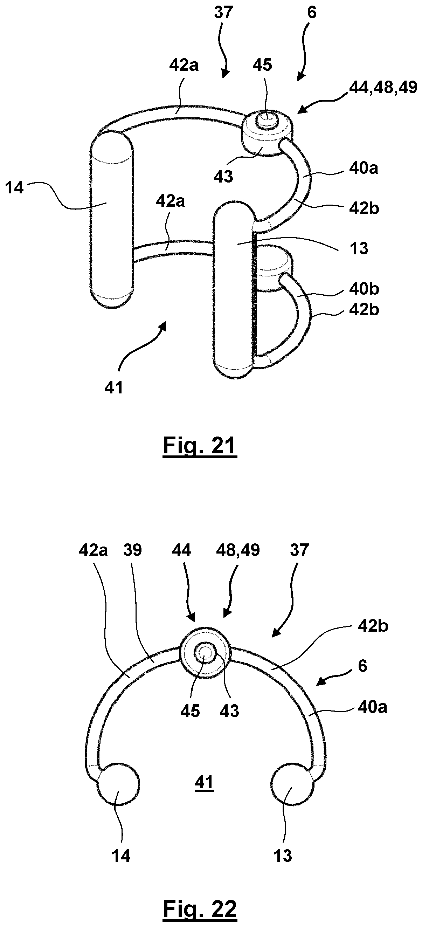

For the embodiment shown in **Figs. 21, 22** the pivot joint 43 comprises a ratchet mechanism 44. The ratchet mechanism 44 allows a movement of the compression structure parts 42a, 42b about the pivot axis in closing direction but generally blocks an opposite movement. Accordingly, a biasing state of the compression structure 37 can be achieved by manually bringing the compression structure parts 42a, 42b into a configuration wherein the compression elements compress the contact areas 5a, 5b. In this case the compression structure parts 42a, 42b are elastically biased by a bending moment. For releasing the compression structure 37 the ratchet mechanism 44 comprises a release button 45. A manipulation of the release button 45 removes the blocking effect of the ratchet mechanism 44 so that it is possible to open the compression structure parts 42a, 42b.

Also for the embodiment shown in **Figs. 21, 22** **the** compression structure 37 comprises two parallel compression structure branches 40a, 40b each comprising two compression structure parts 42a, 42b.

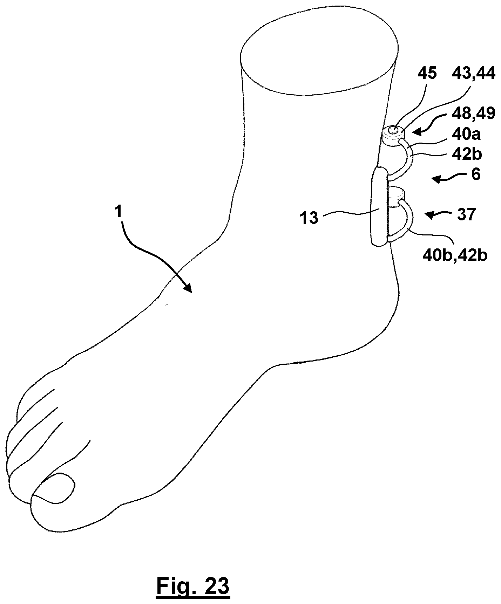

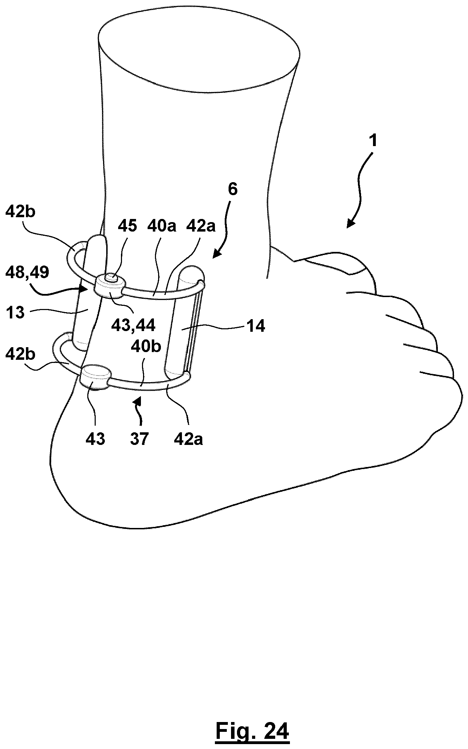

**Figs. 23** **and** **24** show the Achilles tendon orthosis 6 of Figs. 21 and 22 applied to a foot 1.

The Achilles tendon orthosis 6 of the second variant forms a kind of clamp wherein all of the parts comprise an inherent rigidity
a) without any degree of freedom but which is elastically deformed under the compression counterforces 20a, 20b for the embodiment of figs. 17 - 20
b) with only one degree of freedom being blocked by the ratchet mechanism 44 which is elastically deformed under the compression counterforces 20a, 20b for the embodiment of Figs. 21 - 24.

For the variants and embodiments the compression force providing the healing effect is caused by a tightening or elastic bias of the compression structure 37 provided by a biasing mechanism 46:
For the embodiment shown in Fig. 12 the biasing mechanism 46 is formed by the wire 17 and the ratchet mechanism 29 which allows a change of the effective length of the connecting element 11.

For the embodiment of Fig. 13 the biasing mechanism 46 is formed by the strips 17 comprising the hook-and-loop-fasteners 33 allowing a change of the effective length of the connecting element 11.

For the embodiment of Figs. 17 to 20 the biasing mechanism 46 is formed by the elasticity of the compression structure 37, here the compression structure branches 40a, 40b.

For the embodiment shown in Figs. 21 to 24 the biasing mechanism 46 is formed both by the elasticity of the compression structure 37, here the compression structure branches 40a, 40b with the compression structure parts 42a, 42b as well as by the pivot joint 43 comprising the ratchet mechanism 44.

For all of the shown embodiments the biasing mechanism 46 allows a change of the configuration of the Achilles tendon orthosis 6 between
- a non-biasing state wherein the contact surfaces 15, 16 have a distance being larger than or equal to a distance X so that no compression forces are applied to the contact areas 5 of the foot 1 and
- a biasing state wherein the contact surfaces have a distance being smaller than x so that a compression force is applied to the contact areas 5 of the foot 1.

For the embodiments shown in Figs. 12 and 13 the transfer between the non-biasing state and the biasing state is achieved by a change of the effective length of the connecting element 11.

For the embodiment of Figs. 17 to 20 the change from the biasing state to the non-biasing state is achieved by a manual elastic widening of the compression structure 37 leading to an increased bending moment being effective in the compression structure branches 40a, 40b.

For the embodiment of Figs. 21 to 24 a change from the non-biasing state to the biasing state is achieved by pivoting the compression structure parts 42a, 42b towards each other with a ratcheting movement of the ratchet mechanism 44.

It is possible that by a plastic deformation or a thermal treatment the supporting elements 9, 10 and/or the compression structure branches 40 or the compression structure parts 42 are shaped according to the outer contour 8 of the foot 1 so that the shape can be individually adapted to the anatomy of the wearer.

It is possible that a set of components of an Achilles tendon orthosis 6 comprises exchangeable supporting elements 10, 11, connecting elements 11, 12, compression elements 13, 14 and/or compression structure branches 40 or compression structure parts 42 of differing shapes and/or differing stiffnesses for adapting the Achilles tendon orthosis 6 to the specific needs of the wearer. It is also possible that for a variation of the mechanical force characteristics in the Achilles tendon orthosis 6 a different number of compression structure branches 40 can be used. Furthermore, it is possible to individually adapt the length of the compression elements 13, 14 and/or the contact surfaces 15, 16 of the same specifically to the wearer.

It is possible that the compression elements 13, 14 comprise a kind of cushion. These cushions or the compression elements 13, 14 can be made of cellular rubber or foam pads, foam rubber or sponge rubber, PE-foam, PUR-foam, silicone foam, a material distributed under the trademarks "VULKULLAN" or "UREPAN" in cellular foam, latex foam, viton foam, PE-foam, PUR-softfoam, silicone foam, cellular vulkolan, viton foam, evazote, plastazote, silicone, PU, TPU, a gel, a foamed material wherein in particular a material plastazote LD60 with a volumetric weight of 60 kg/m³ is used. As a material for the components having an inherent rigidity the materials PA, PE, PVC, deformable hard-foam plates made of PE, PA, PVC and materials that can be deformed by the application of heat, metal sheets made of aluminum or any other metal or also any plastic material might be used.

In the present application for a simplification it is assumed that the Achilles tendon 4 has a vertical orientation and a straight extension. The skilled person will know that this is not exactly the case. Accordingly, for orientations given in the present application (in particular the orientation Y of the Achilles tendon 4 and a plane or orientation perpendicular to the orientation Y) also orientations are covered differing from the mentioned orientations by an angle of less than 30°, less than 20°, less than 10° or less than 5°.

An inventive Achilles tendon orthosis 6 might comprise an adjusting mechanism 48. By means of the adjusting mechanism 48 it is possible to adjust at least one dimension and/or a shape of the compression structure 37. For the embodiment of Figs. 3, 12, 13, 15 the adjusting mechanism 48 changes the effective length of the wire, thread, strip or filament 17. Instead, for the embodiment of Figs. 21 - 24 the adjusting mechanism 48 is formed by the pivot joint 43 with the ratchet mechanism 44 allowing a change of the shape of the compression structure 37 by changing the angle between the compression structure parts 42a, 42b. In this embodiment the adjusting mechanism 48 is a closing mechanism 48 allowing a closure of the compression structure parts 42a, 42b with a securing of a reached closed state by the ratchet mechanism 44.

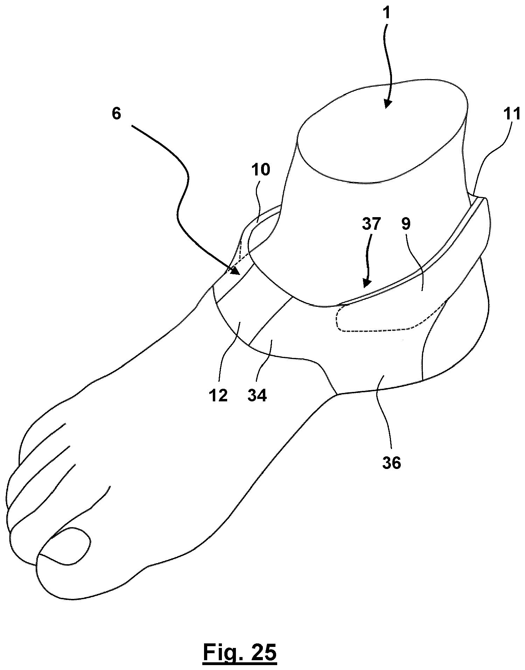

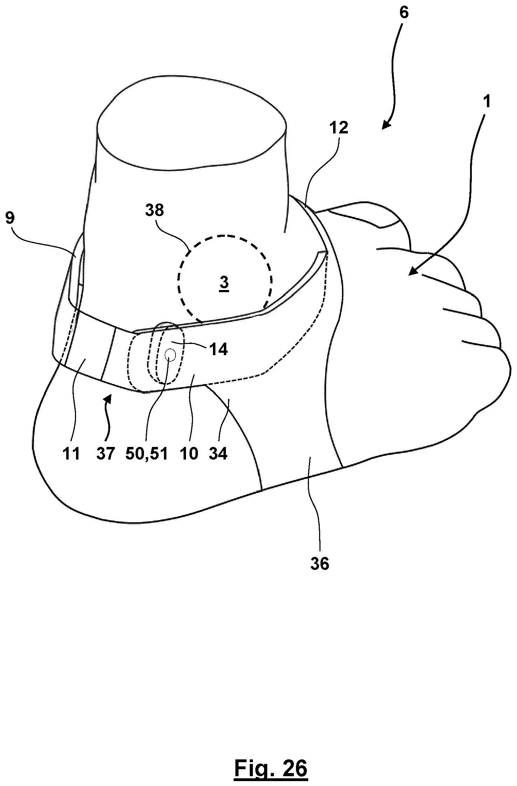

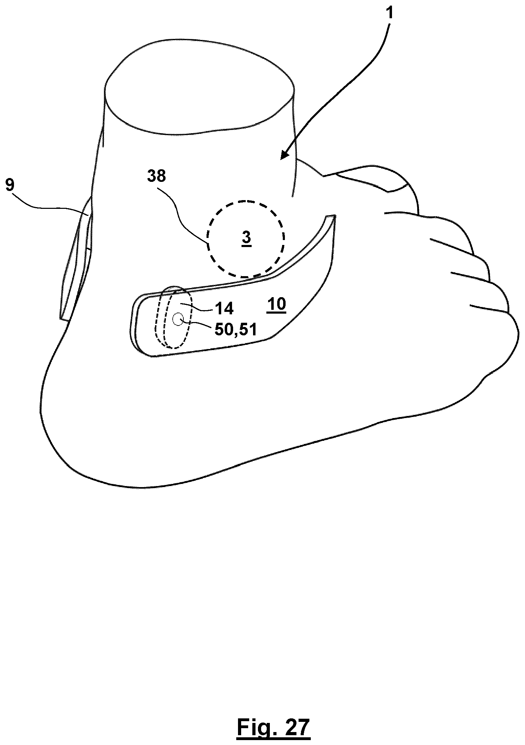

Figs. 25 to 27 show another embodiment of an Achilles tendon orthosis 6. Whereas for the former embodiments the compression structure 37 (in particular the supporting elements 9, 10 or the compression structure branches 40a, 40b) extended in heights above and below the convex curvature 38 of the ankle joint 3, for the embodiment of Figs. 25 to 27 the Achilles tendon orthosis 6 (here the cover 34, supporting elements 9, 10 and connecting elements 11, 12 as well as a pulling stirrup 36 integrally formed by the cover 34) extend below the convex curvature 38 of the ankle joint 3. Furthermore, here the compression elements 13, 14 have an extension parallel to the orientation Y of the Achilles tendon 4 which is smaller than the extension for the former embodiments, in particular in the range of 1 - 4 cm or 1,5 - 3,0 cm. Preferably, over the entire longitudinal extension the compression elements 13, 14 are covered by the supporting elements 9, 10.

Furthermore, for the embodiment shown in Figs. 25 to 27 the compression elements 13, 14 are not fixed to the compression structure 37, here the supporting elements 9, 10. Instead, the compression elements 13, 14 are connected to the associated supporting element 9, 10 by a guidance 50. The guidance 50 provides at least one degree of freedom allowing a relative movement between the compression element 13, 14 and the associated supporting element 9, 10. This degree of freedom might be a translational and/or rotational degree of freedom. For the shown embodiment the guidance 50 is provided by a pivot joint 51 which is here embodied by a pivot bolt held by the supporting element 9, 10 (or held by the compression element 13, 14) and being accommodated in a bearing lug of the compression element 13, 14 (or in the supporting element 10, 11). For this embodiment the guidance 50 allows an adjusting or compensating pivoting relative movement which in particular provides that when walking or running with the applied Achilles tendon orthosis 6 accompanied by a pivoting movement of the ankle joint 3 the compression elements 13, 14 are pressed against the contact area 5 without a slipping movement occurring in the contact area 5.

Also for the other embodiments shown and described the compression elements 13, 14 might not be fixed to the supporting elements 9, 10 but connected to the supporting elements 9, 10 or compression structure 37 by a guidance 50 or pivot joint 51.

For all of the disclosed embodiments it is possible that the connecting elements 11, 12
a) have a rigidity such that the connecting elements 11, 12 do not undergo considerable elastic elongations under the effective pulling forces or
b) have an elasticity such that the connecting elements 11, 12 undergo considerable elastic elongations under the effective pulling forces so that the connecting elements 11, 12 are elastically stretched in the applied state of the Achilles tendon orthosis 6.

According to the invention it is possible to adjust the compression force applied by the Achilles tendon orthosis 6 to the wearer. This might be achieved by use of a manual actuation element 52, an actuation wheel 30, a ratchet mechanism 29 or an adjusting mechanism 48 and it is possible that the compression force is adjusted in at least two steps, a variety of steps or in a continuous fashion. It is also possible that the compression force is adjusted by a tensioning belt, a hook-and-loop fastener and by other adjustable pulling means.

It is furthermore possible that the Achilles tendon orthosis 6 is part of a bandage, a frame construction or a kind of shoe.

The inventive Achilles tendon orthosis applies a dosed compression force upon the affected area where the hypervascularity occurs in the Achilles tendon region. This leads to a kind of "sclerotization" of the effected blood vessels.

The use of the inventive Achilles tendon orthosis is a pure conservative measure which might be used without any surgical invention or without the implication of a required injection.

It is possible that the compression elements 13, 14 and/or other parts of the Achilles tendon orthosis 6 are manufactured by 3D-printing from any plastic material or other materials.

### LIST OF REFERENCE NUMERALS

- 1: foot
- 2: lower leg
- 3: ankle joint
- 4: Achilles tendon
- 5: contact area
- 6: Achilles tendon orthosis
- 7: ring
- 8: outer contour
- 9: supporting element
- 10: supporting element
- 11: connecting element
- 12: connecting element
- 13: compression element
- 14: compression element
- 15: contact surface
- 16: contact surface
- 17: wire, thread, strip or filament
- 18: intermediate space
- 19: pulling force
- 20: compression counterforce
- 21: pulling force
- 22: base leg
- 23: side leg
- 24: side leg
- 25: distance
- 26: weakening
- 27: groove
- 28: plane
- 29: ratchet mechanism
- 30: actuation wheel
- 31: eye
- 32: tackle
- 33: hook-and-loop-fasteners
- 34: cover
- 35: strip
- 36: pulling stirrup
- 37: compression structure
- 38: convex curvature
- 39: bracket, C, V or U
- 40: compression structure branch
- 41: opening
- 42: compression structure part
- 43: pivot joint
- 44: ratchet mechanism
- 45: release button
- 46: biasing mechanism
- 47: compression structure plane
- 48: adjusting mechanism
- 49: closing mechanism
- 50: guidance
- 51: pivot joint
- 52: manual actuation element

## Claims

1. Achilles tendon orthosis (6)
designated for treating an Achilles tendon (4) of a foot (1),
- the Achilles tendon (4) having an orientation Y,
- the foot (1) in a cross section taken perpendicular to the orientation Y and taken through the Achilles tendon (4) having an outer contour (8),
- the outsides of the skin parts of the foot (1) covering the Achilles tendon (4) having a distance X,
the Achilles tendon orthosis (6) comprising
a) a compression structure (37) extending in a compression structure plane (47) having an orientation perpendicular to the orientation Y and
b) two compression elements (13, 14) forming elongate opposing contact surfaces (15, 16) having an orientation parallel to the orientation Y, the compression elements (13, 14) being supported by the compression structure (37) and being designated for contacting the foot in contact areas (5),
c) a biasing mechanism (46) which allows a change of the configuration of the Achilles tendon orthosis (6) between
ca) a non-biasing state wherein the contact surfaces (15, 16) have a distance being larger than or equal to X so that when applying the Achilles tendon orthosis (6) the compression elements (13, 14) do not apply a compression force to the contact areas (4) and
cb) a biasing state wherein the contact surfaces (15, 16) have a distance being smaller than X when no compression counterforces (20) are applied to the contact surface (15, 16) of the compression elements (13, 14) so that when applying the Achilles tendon orthosis (6) the compression elements (13, 14) apply a compression force to the contact areas (4),
d) the Achilles tendon orthosis (6) being dimensioned, configured and designed such that when applying the Achilles tendon orthosis (6) to a foot (1) the contact surfaces (15, 16) of the compression elements (13, 14) contact the foot (1) at a location anterior to the mid-portion Achilles tendon and posterior to the lower leg (2) and/or ankle joint (3) and
**characterized in that**
e) the compression structure (37) comprises supporting elements (9, 10) having an inherent rigidity, the supporting elements (9, 10) support the compression elements (13, 14) and by compression counterforces (20) acting upon the contact surfaces (15, 16) of the compression elements (13, 14) the supporting elements (9, 10) are biased by a bending moment.

2. Achilles tendon orthosis (6) of claim 1, **characterized in that** the Achilles tendon orthosis (6) is dimensioned, configured and designed such that when applying the Achilles tendon orthosis (6) to a foot (1) the compression structure (15, 16) does not contact the foot (1) along the whole circumference but delimits at least one intermediate space (18) between the compression structure (37) and the foot (1).

3. Achilles tendon orthosis (6) of claim 1 or 2, **characterized in that** the compression elements (13, 14) and/or the contact surfaces (15, 16) have a length in the range of 4 cm to 8 cm.

4. Achilles tendon orthosis (6) of one of claims 1 to 3, **characterized in that**
a) the positions where the compression elements (13, 14) are supported by the compression structure (37) and/or
b) the angles between the longitudinal axes of the compression elements (13, 14) and the compression structure (37)
are adjustable.

5. Achilles tendon orthosis (6) of one of claims 1 to 4, **characterized in that** at least one of the supporting elements (9, 10) is H-, V- or U-shaped.

6. Achilles tendon orthosis (6) of one of claims 1 to 5, **characterized in that** a part or portion of the compression structure (37) having an inherent rigidity comprises a local weakening (26).

7. Achilles tendon orthosis (6) of one of claims 1 to 6, **characterized in that** a cover (34) is provided that covers, supports and/or embeds the compression structure (37) and/or the compression elements (13, 14).

8. Achilles tendon orthosis (6) of one of claims 1 to 7, **characterized in that** a loop-shaped pulling stirrup (36) is provided which connects one supporting element (9) to the other supporting element (10).

9. Achilles tendon orthosis (6) of one of claims 1 to 8, **characterized in that** the compression elements (13, 14) are supported by the compression structure (37) by a guidance (50), the guidance (50) providing at least one degree of freedom for allowing a relative movement between the compression elements (13, 14) and the compression structure (37).

10. Achilles tendon orthosis (6) of one of claims 1 to 9, **characterized in that** a manual actuation element (52) is provided, a manual actuation of the manual actuation element (52) leading to an increase or decrease of the compression force.

11. Achilles tendon orthosis (6) of one of claims 1 to 10, **characterized in that**
a) the compression structure (37) forms a closed ring (7) extending in the compression structure plane,
b) the ring (7) comprises two supporting elements (9, 10)
ba) having an inherent rigidity and
bb) each supporting one of the compression elements (13, 14) at a location between the end portions of the supporting element (9, 10),
c) one end portion of a first supporting element (9) is connected by a first connecting element (11) to an end portion of the second supporting element (10),
d) the other end portion of the first supporting element (9) is connected by a second connecting element (12) to the other end portion of the second supporting element (10),
e) the biasing mechanism (46) is formed by an adjustable effective length of at least one connecting element (11, 12).

12. Achilles tendon orthosis (6) of claim 11, **characterized in that**
a) at least one connecting element (11, 12) is formed with a wire, thread, strip or filament (17) and a ratchet mechanism (29) and the effective length of the wire, thread, strip or filament (17) is adjusted by a manipulation of the ratchet mechanism (29), the wire, thread, strip or filament (17) in particular forming a tackle (32) or
b) at least one of the connecting elements (11, 12) comprises at least one strip (17) having a hook-and-loop-fastener (33) or mushroom-and-loop-fastener for adjusting the effective length.

13. Achilles tendon orthosis (6) of one of claims 1 to 12, **characterized in that**
a) the compression structure (37) is bracket-shaped, C-shaped, V-shaped or U-shaped
b) the compression structure (37) and/or the compression elements (13, 14) are/is elastic,
c) in the non-biasing state of the Achilles tendon orthosis (6) the elastic bias of the compression structure (37) and/or the compression elements (13, 14) is higher than in the biasing state of the Achilles tendon orthosis (6),
d) the compression elements (13, 14) are supported at the end portions of the compression structure (37),
e) the compression structure (37) in particular comprising two parallel compression structure branches (40a, 40b) being connected in their end regions to the compression elements (13, 14).

14. Achilles tendon orthosis (6) of claim 13, **characterized in that** an adjusting mechanism (48) is provided which upon manipulation adjusts at least one dimension and/or shape of the compression structure (37).

15. Achilles tendon orthosis (6) of one of claims 13 to 14, **characterized in that**
a) the compression structure (37) or the compression structure branches (40a, 40b) comprises or comprise two compression structure parts (42a, 42b) being connected to each other in neighboring end regions by a joint (43) defining a pivot axis and being connected to an associated compression elements (13, 14) in the other end region,
b) a closing mechanism (49) is provided
ba) the closing mechanism (49) comprising a ratchet mechanism (44) which allows a pivoting movement of the compression structure parts (42a, 42b) about the pivot axis in closing direction but blocks a pivoting movement of the compression structure parts (42a, 42b) about the pivot axis in opening direction or
bb) the closing mechanism (4) comprising a spring which biases the compression structure parts (42a, 42b) for a pivoting movement about the pivot axis in closing direction.

## Patentansprüche

1. Achillessehnenorthese (6),
die für eine Behandlung einer Achillessehne (4) eines Fußes (1) bestimmt ist, wobei
- die Achillessehne (4) einer Orientierung Y hat,
- der Fuß (1) in einem Querschnitt, der vertikal zu der Orientierung Y und durch die Achillessehne (4) verläuft, eine äußere Kontur (8) hat,
- die Außenseiten der Hautteile des Fußes (1), die die Achillessehne (4) überdecken, einen Abstand X haben,
wobei die Achillessehnenorthese (6)
a) eine Kompressionsstruktur (37), die sich in einer Kompressionsstrukturebene (47) erstreckt, die eine Orientierung senkrecht zu der Orientierung Y aufweist, und
b) zwei Kompressionselemente (13, 14), die längliche gegenüberliegende Kontaktflächen (15, 16) mit einer Orientierung parallel zu der Orientierung Y bilden, wobei die Kompressionselemente (13, 14) von der Kompressionsstruktur (37) getragen oder abgestützt sind und für die Kontaktierung des Fußes in Kontaktbereichen (5) bestimmt sind,
c) einen Beaufschlagungsmechanismus (46), der eine Veränderung der Konfiguration der Achillessehnenorthese (6) ermöglicht zwischen
ca) einem nicht-beaufschlagenden Zustand, in dem die Kontaktflächen (15, 16) einen Abstand haben, der größer oder gleich X ist, so dass bei Applikation der Achillessehnenorthese (6) die Kompressionselemente (13, 14) keine Kompressionskraft auf die Kontaktbereiche (4) ausüben, und
cb) einem beaufschlagenden Zustand, in dem die Kontaktflächen (15, 16) einen Abstand haben, der kleiner ist als X, wenn keine Kompressions-Gegenkräfte (20) auf die Kontaktflächen (15, 16) der Kompressionselemente (13, 14) aufgebracht werden, so dass bei der Applikation der Achillessehnenorthese (6) die Kompressionselemente (13, 14) eine Kompressionskraft auf die Kontaktbereiche (4) ausüben,
aufweist,
d) wobei die Achillessehnenorthese (6) so dimensioniert, konfiguriert und gestaltet ist, dass bei Applikation der Achillessehnenorthese (6) an einen Fuß (1) die Kontaktflächen (15, 16) der Kompressionselemente (13, 14) den Fuß (1) an einem Ort anterior zu dem Mittenabschnitt der Achillessehne und posterior zu dem Unterschenkel (2) und/oder dem Fuß- oder Sprunggelenk (3) kontaktieren, und
**dadurch gekennzeichnet, dass**
e) die Kompressionsstruktur (37) Trag- oder Stützelemente (9, 10) aufweist, die eine inhärente Steifigkeit haben, wobei die Trag- oder Stützelemente (9, 10) die Kompressionselemente (13, 14) tragen oder abstützen, und die Trag- oder Stützelemente (9, 10) durch die Kompressions-Gegenkräfte (20), die auf die Kontaktflächen (15, 16) der Kompressionselemente (13, 14) wirken, mit einem Biegemoment beaufschlagt sind.

2. Achillessehnenorthese (6) nach Anspruch 1, **dadurch gekennzeichnet, dass** die Achillessehnenorthese (6) derart dimensioniert, konfiguriert und gestaltet ist, dass bei Applikation der Achillessehnenorthese (6) an einen Fuß (1) die Kompressionsstruktur (15, 16) den Fuß (1) nicht entlang des gesamten Umfangs kontaktiert, sondern zumindest einen Zwischenraum (18) zwischen der Kompressionsstruktur (37) und dem Fuß (1) begrenzt.

3. Achillessehnenorthese (6) nach Anspruch 1 oder 2, **dadurch gekennzeichnet, dass** die Kompressionselemente (13, 14) und/oder die Kontaktflächen (15, 16) eine Länge im Bereich von 4 cm bis 8 cm haben.

4. Achillessehnenorthese (6) nach einem der Ansprüche 1 bis 3, **dadurch gekennzeichnet, dass**
a) die Positionen, an denen die Kompressionselemente (13, 14) durch die Kompressionsstruktur (37) getragen oder abgestützt sind, und/oder
b) die Winkel zwischen den Längsachsen der Kompressionselemente (13, 14) und der Kompressionsstruktur (37)
einstellbar sind.

5. Achillessehnenorthese (6) nach einem der Ansprüche 1 bis 4, **dadurch gekennzeichnet, dass** mindestens eines der Trag- oder Stützelemente (9, 10) H-, V- oder U-förmig ist.

6. Achillessehnenorthese (6) nach einem der Ansprüche 1 bis 5, **dadurch gekennzeichnet, dass** ein Teil oder Abschnitt der Kompressionsstruktur (37), welcher oder welche eine inhärente Steifigkeit aufweist, eine lokale Schwächung (26) aufweist.

7. Achillessehnenorthese (6) nach einem der Ansprüche 1 bis 6, **dadurch gekennzeichnet, dass** eine Abdeckung (34) vorhanden ist, welche die Kompressionsstruktur (37) und/oder die Kompressionselemente (13, 14) abdeckt, stützt, trägt und/oder einbettet.

8. Achillessehnenorthese (6) nach einem der Ansprüche 1 bis 7, **dadurch gekennzeichnet, dass** ein ring- oder schlaufenförmiger Zugbügel (36) vorhanden ist, welcher ein Trag- oder Stützelement (9) mit dem anderen Trag- oder Stützelement (10) verbindet.

9. Achillessehnenorthese (6) nach einem der Ansprüche 1 bis 8, **dadurch gekennzeichnet, dass** die Kompressionselemente (13, 14) mittels einer Führung (50) von der Kompressionsstruktur (37) getragen oder abgestützt sind, wobei die Führung (50) mindestens einen Freiheitsgrad bereitstellt zur Ermöglichung einer Relativbewegung zwischen den Kompressionselementen (13, 14) und der Kompressionsstruktur (37).

10. Achillessehnenorthese (6) nach einem der Ansprüche 1 bis 9, **dadurch gekennzeichnet, dass** ein manuelles Betätigungselement (52) vorhanden ist, wobei eine manuelle Betätigung des manuellen Betätigungselements (52) zu einer Vergrößerung oder Verkleinerung der Kompressionskraft führt.

11. Achillessehnenorthese (6) nach einem der Ansprüche 1 bis 10, **dadurch gekennzeichnet, dass**
a) die Kompressionsstruktur (37) einen geschlossenen Ring (7) bildet, der sich in der Kompressionsstrukturebene erstreckt,
b) der Ring (7) zwei Trag- oder Stützelemente (9, 10) aufweist, die
ba) eine inhärente Steifigkeit haben und
bb) jeweils eines der Kompressionselemente (13, 14) an einem Ort zwischen den Endbereichen des Trag- oder Stützelements (9, 10) tragen oder stützen,
c) wobei ein Endbereich eines ersten Trag- oder Stützelements (9) durch ein erstes Verbindungselement (11) mit einem Endbereich des zweiten Trag- oder Stützelements (10) verbunden ist,
d) der andere Endbereich des ersten Trag- oder Stützelements (9) durch ein zweites Verbindungselement (12) mit dem anderen Endbereich des zweiten Trag- oder Stützelements (10) verbunden ist,
e) der Beaufschlagungsmechanismus (46) durch eine einstellbare wirksame Länge mindestens eines Verbindungselements (11, 12) gebildet ist.

12. Achillessehnenorthese (6) nach Anspruch 11, **dadurch gekennzeichnet, dass**
a) mindestens ein Verbindungselement (11, 12) mit einem Draht, Faden, Streifen oder Filament (17) und einem Ratschenmechanismus (29) gebildet ist und die effektive Länge des Drahtes, Fadens, Streifens oder Filaments (17) ist durch eine Manipulation des Ratschenmechanismus (29) einstellbar, wobei der Draht, Faden, Streifen oder das Filament (17) insbesondere einen Flaschen- oder Seilzug (32) bildet oder
b) mindestens eines der Verbindungselemente (11, 12) mindestens einen Streifen (17) mit einem Klettverschlusselement (33) für eine Anpassung der effektiven Länge aufweist.

13. Achillessehnenorthese (6) nach einem der Ansprüche 1 bis 12, **dadurch gekennzeichnet, dass**
a) die Kompressionsstruktur (37) leisten-, bogen- oder klammerförmig, C-förmig, V-förmig oder U-förmig ist,
b) die Kompressionsstruktur (37) und/oder die Kompressionselemente (13, 14) elastisch ist/sind,
c) in dem nicht beaufschlagenden Zustand der Achillessehnenorthese (6) die elastische Beaufschlagung der Kompressionsstruktur (37) und/oder der Kompressionselemente (13, 14) größer ist als in dem beaufschlagenden Zustand der Achillessehnenorthese (6),
d) die Kompressionselemente (13, 14) an den Endbereichen der Kompressionsstruktur (37) getragen oder abgestützt sind,
e) wobei die Kompressionsstruktur (37) insbesondere zwei parallele Kompressionsstrukturzweige (40a, 40b) aufweist, in ihren Endbereichen mit den Kompressionselementen (13, 14) verbunden ist.

14. Achillessehnenorthese (6) nach Anspruch 13, **dadurch gekennzeichnet, dass** ein Einstellmechanismus (48) vorhanden ist, der bei Manipulation mindestens eine Dimension und/oder Form der Kompressionsstruktur (37) einstellt.

15. Achillessehnenorthese (6) nach einem der Ansprüche 13 bis 14, **dadurch gekennzeichnet, dass**
a) die Kompressionsstruktur (37) oder die Kompressionsstrukturzweige (40a, 40b) zwei Kompressionsstrukturteile (42a, 42b) aufweist oder aufweisen, die in benachbarten Endbereichen mittels eines Gelenks (43), welches eine Schwenkachse definiert, miteinander verbunden sind und in dem anderen Endbereich mit einem zugeordneten Kompressionselement (13, 14) verbunden sind,
b) wobei ein Schließmechanismus (49) vorhanden ist,
ba) wobei der Schließmechanismus (49) einen Ratschenmechanimus (44) aufweist, der eine Schwenkbewegung der Kompressionsstrukturteile (42a, 42b) um die Schwenkachse in Schließrichtung ermöglicht, aber eine Schwenkbewegung der Kompressionsstrukturteile (42a, 42b) um die Schwenkachse in Öffnungsrichtung blockiert, oder
bb) wobei der Schließemechanismus (4) eine Feder aufweist, die die Kompressionsstrukturteile (42a, 42b) für eine Schwenkbewegung in Schließrichtung um die Schwenkachse beaufschlagt.

## Revendications

1. Orthèse de tendon d'Achille (6)
destinée à traiter un tendon d'Achille (4) d'un pied (1),
- le tendon d'Achille (4) ayant une orientation Y,
- le pied (1) selon une coupe transversale prise perpendiculairement à l'orientation Y et prise à travers le tendon d'Achille (4) ayant un contour externe (8),
- les parties extérieures des parties cutanées du pied (1) recouvrant le tendon d'Achille (4) ayant une distance X,
l'orthèse de tendon d'Achille (6) comprenant
a) une structure de compression (37) s'étendant dans un plan de structure de compression (47) ayant une orientation perpendiculaire à l'orientation Y et
b) deux éléments de compression (13, 14) formant des surfaces de contact opposées allongées (15, 16) ayant une orientation parallèle à l'orientation Y, les éléments de compression (13, 14) étant supportés par la structure de compression (37) et étant destinés à être en contact avec le pied dans des zones de contact (5),
c) un mécanisme de sollicitation (46) qui permet un changement de la configuration de l'orthèse de tendon d'Achille (6) entre
ca) un état de non-sollicitation dans lequel les surfaces de contact (15, 16) ont une distance supérieure ou égale à X de telle sorte que lors de l'application de l'orthèse de talon d'Achille (6), les éléments de compression (13, 14) n'appliquent pas une force de compression sur les zones de contact (4),
cb) un état de sollicitation dans lequel les surfaces de contact (15, 16) ont une distance inférieure à X lorsqu'aucune force de compression contraire (20) n'est appliquée à la surface de contact (15, 16) des éléments de compression (13, 14) de telle sorte que lors de l'application de l'orthèse de tendon d'Achille (6), les éléments de compression (13, 14) appliquent une force de compression sur les zones de contact (4),
d) l'orthèse de tendon d'Achille (6) étant dimensionnée, configurée et conçue de telle sorte que lors de l'application de l'orthèse de talon d'Achille (6) à un pied (1) les surfaces de contact (15, 16) des éléments de compression (13, 14) entrent en contact avec le pied (1) au niveau d'un emplacement antérieur à la portion médiane du tendon d'Achille et postérieur à la jambe (2) et/ou à l'articulation de la cheville (3) et
**caractérisée en ce que**
e) la structure de compression (37) comprend des éléments de support (9, 10) ayant une rigidité inhérente, les éléments de support (9, 10) supportent les éléments de compression (13, 14) et sous l'effet de forces contraires de compression (20) agissant sur les surfaces de contact (15, 16) des éléments de compression (13, 14) les éléments de support (9, 10) sont sollicités par un moment de flexion.

2. Orthèse de tendon d'Achille (6) selon la revendication 1, **caractérisée en ce que** l'orthèse de tendon d'Achille (6) est dimensionnée, configurée et conçue de telle sorte que lors de l'application de l'orthèse de tendon d'Achille (6) à un pied (1), la structure de compression (15, 16) n'entre pas en contact avec le pied (1) le long de l'ensemble de la circonférence mais délimite au moins un espace intermédiaire (18) entre la structure de compression (37) et le pied (1).

3. Orthèse de tendon d'Achille (6) selon la revendication 1 ou 2, **caractérisée en ce que** les éléments de compression (13, 14) et/ou les surfaces de contact (15, 16) ont une longueur dans la plage de 4 cm à 8 cm.

4. Orthèse de tendon d'Achille (6) selon l'une des revendications 1 à 3, **caractérisée en ce que**
a) les positions où lesquelles les éléments de compression (13, 14) sont supportés par la structure de compression (37) et/ou
b) les angles entre les axes longitudinaux des éléments de compression (13, 14) et la structure de compression (37)
sont réglables.

5. Orthèse de tendon d'Achille (6) selon l'une des revendications 1 à 4, **caractérisée en ce qu'**au moins l'un des éléments de support (9, 10) est en forme de H, de V ou de U.

6. Orthèse de tendon d'Achille (6) selon l'une des revendications 1 à 5, **caractérisée en ce qu'**une partie ou une portion de la structure de compression (37) ayant une rigidité inhérente comprend une fragilité locale (26).

7. Orthèse de tendon d'Achille (6) selon l'une des revendications 1 à 6, **caractérisée en ce qu'**il est prévu une protection (34) qui couvre, supporte et/ou intègre la structure de compression (37) et/ou les éléments de compression (13, 14).

8. Orthèse de tendon d'Achille (6) selon l'une des revendications 1 à 7, **caractérisée en ce qu'**il est prévu un étrier de traction en forme de boucle (36) qui relie un élément de support (9) à l'autre élément de support (10).

9. Orthèse de tendon d'Achille (6) selon l'une des revendications 1 à 8, **caractérisée en ce que** les éléments de compression (13, 14) sont supportés par la structure de compression (37) par un guidage (50), le guidage (50) conférant au moins un degré de liberté pour permettre un mouvement relatif entre les éléments de compression (13, 14) et la structure de compression (37).

10. Orthèse de tendon d'Achille (6) selon l'une des revendications 1 à 9, **caractérisée en ce qu'**il est prévu un élément d'actionnement manuel (52), un actionnement manuel de l'élément d'actionnement manuel (52) provoquant une augmentation ou diminution de la force de compression.

11. Orthèse de tendon d'Achille (6) selon l'une des revendications 1 à 10, **caractérisée en ce que**
a) la structure de compression (37) forme un anneau fermé (7) s'étendant dans le plan de la structure de compression,
b) l'anneau (7) comprend deux éléments de support (9, 10)
ba) ayant une rigidité inhérente et
bb) chacun supportant l'un des éléments de compression (13, 14) au niveau d'un emplacement entre les portions d'extrémité de l'élément de support (9, 10),
c) une portion d'extrémité d'un premier élément de support (9) est reliée par un premier élément de liaison (11) à une portion d'extrémité du deuxième élément de support (10),
d) l'autre portion d'extrémité du premier élément de support (9) est reliée par un deuxième élément de liaison (12) à l'autre portion d'extrémité du deuxième élément de support (10),
e) le mécanisme de sollicitation (46) est formé par une longueur effective réglable d'au moins un élément de liaison (11, 12).

12. Orthèse de tendon d'Achille (6) selon la revendication 11, **caractérisée en ce que**
a) au moins un élément de liaison (11, 12) est formé avec un câble, un fil, une bande ou un filament (17) et un mécanisme à cliquet (29) et la longueur effective du câble, du fil, de la bande ou du filament (17) est réglée par une manipulation du mécanisme à cliquet (29), le câble, le fil, la bande ou le filament (17) formant en particulier un palan (32) ou
b) au moins l'un des éléments de liaison (11, 12) comprend au moins une bande (17) ayant une fermeture auto-agrippante (33) ou un auto-agrippant de type champignon pour régler la longueur effective.

13. Orthèse de tendon d'Achille (6) selon l'une des revendications 1 à 12, **caractérisée en ce que**
a) la structure de compression (37) est en forme de parenthèse, en forme de C, en forme de V ou en forme de U
b) la structure de compression (37) et/ou les éléments de compression (13, 14) est/sont élastique(s),
c) dans l'état de non sollicitation de l'orthèse de tendon d'Achille (6) la sollicitation élastique de la structure de compression (37) et/ou des éléments de compression (13, 14) est plus élevée que dans l'état de sollicitation de l'orthèse de tendon d'Achille (6),
d) les éléments de compression (13, 14) sont supportés au niveau des portions d'extrémité de la structure de compression (37),
e) la structure de compression (37) comprenant en particulier deux branches de structure de compression parallèles (40a, 40b) étant reliées dans leurs régions d'extrémité aux éléments de compression (13, 14).

14. Orthèse de tendon d'Achille (6) selon la revendication 13, **caractérisée en ce qu'**il est prévu un mécanisme de réglage (48) qui, lorsqu'il est manipulé, règle au moins une dimension et/ou forme de la structure de compression (37).

15. Orthèse de tendon d'Achille (6) selon l'une des revendications 13 à 14, **caractérisée en ce que**
a) la structure de compression (37) ou les branches de la structure de compression (40a, 40b) comprend ou comprennent deux parties de structure de compression (42a, 42b) étant reliées l'une à l'autre dans des régions d'extrémité voisines par une articulation (43) définissant un axe de pivotement et étant reliées à un des éléments de compression (13, 14) associé dans l'autre région d'extrémité,
b) il est prévu un mécanisme de fermeture (49)
ba) le mécanisme de fermeture (49) comprenant un mécanisme à cliquet (44) qui permet un mouvement de pivotement des parties de structure de compression (42a, 42b) autour de l'axe de pivotement dans la direction de fermeture mais bloque un mouvement de pivotement des parties de structure de compression (42a, 42b) autour de l'axe de pivotement dans la direction d'ouverture ou
bb) le mécanisme de fermeture (4) comprenant un ressort qui sollicite les parties de structure de compression (42a, 42b) pour un mouvement de pivotement autour de l'axe de pivotement dans la direction de fermeture.
